# EUROPÄISCHE PATENTSCHRIFT

(11) **EP 4 154 973 B1**
(45) Veröffentlichungstag und Bekanntmachung des Hinweises auf die Patenterteilung: **17.01.2024**
(21) Anmeldenummer: 21199464.5
(22) Anmeldetag: 28.09.2021
(51) Int. Cl.: B01F 35/71, B01F 35/75, B01F 33/501, A61B 17/88, B01F 101/20

(54) **VORRICHTUNG ZUM BEREITSTELLEN VON KNOCHENZEMENTTEIG**
DEVICE FOR PREPARING BONE CEMENT PASTE
DISPOSITIF DE PRÉPARATION DE PÂTE DE CIMENT OSSEUX

(43) Veröffentlichungstag der Anmeldung: 29.03.2023
(73) Patentinhaber: Heraeus Medical GmbH, 61273 Wehrheim (DE)
(72) Erfinder: VOGT, Sebastian, 61273 Wehrheim (DE); KLUGE, Thomas, 61273 Wehrheim (DE)
(74) Vertreter: Heraeus IP

(56) Entgegenhaltungen:
- EP-A1- 3 384 866
- EP-A1- 3 395 274
- DE-A1- 4 409 610
- US-A- 4 735 509
- US-A1- 2006 274 601

## Beschreibung

Vorrichtung zum Bereitstellen eines Knochenzementteigs aus zwei Ausgangskomponenten, umfassend eine hohlzylinderförmige Kartusche mit einem Innenraum, wobei in einem proximalen Teil des Innenraums ein Knochenzementpulver als erste Ausgangskomponente und in einem distalen Teil des Innenraums mindestens zwei Beutel enthaltend eine Monomerflüssigkeit als zweite Ausgangskomponente lagert,
wobei zwischen dem Knochenzementpulver und den mindestens zwei Beuteln ein axial im Innenraum beweglicher Austragskolben und auf einer dem Austragskolben axial gegenüberliegenden Seite der mindestens zwei Beutel ein axial im Innenraum beweglicher Förderkolben angeordnet ist,
wobei der proximale Teil und der distale Teil des Innenraums über ein Leitungsmittel fluidleitend miteinander verbunden sind.

Die Erfindung betrifft weiterhin ein Verfahren zum Bereitstellen eines Knochenzementteigs aus zwei Ausgangskomponenten mittels einer derartigen Vorrichtung.

### Hintergrund der Erfindung

Es werden erhebliche Bemühungen unternommen, Vorrichtungen und Verfahren zum Bereitstellen von Knochenzement aufzuzeigen, mittels derer Knochenzementteig einfach, zuverlässig und schnell bereitgestellt werden kann. Ein wichtiger Aspekt bei der Bereitstellung von Knochenzementteig ist die Vermeidung von Lufteinschlüssen, wie beispielsweise Gasblasen, im Knochenzement. Zu deren Vermeidung wurden eine Vielzahl von Vakuum-Zementiersystemen beschreiben, von denen exemplarisch folgende genannt sind:
US 6,033,105 A, US 5,624,184 A, US 4,671,263 A, US 4,973,168 A, US 5,100,241 A, WO 99/67015 A1, EP 1020167 A2, US 5,586,821 A, EP 1016452 A2, DE 3640279 A1, WO 94/26403 A1, EP 1005901 A2, EP 1886647 A1, US 5,344,232 A.

Es besteht im Markt der Wunsch zur Vereinfachung der Bereitstellung von Knochenzementteig. Eine Weiterentwicklung besteht in der Entwicklung von Zementiersystemen, in denen beide Ausgangskomponenten in separaten Bereichen der Mischsysteme gelagert sind und erst unmittelbar vor der Zementierapplikation im Zementiersystem miteinander vermischt werden. Solche geschlossenen, sogenannten Full-Prepacked-Systeme, sind in folgenden Schriften genannt: EP 0 692 229 A1, DE 10 2009 031 178 B3, US 5,997,544 A, US 6,709,149 B1, DE 698 12 726 T2, EP 0 796 653 A2, US 5,588,745 A.

In den vorgenannten Full-Prepacked-Systemen erfolgt die Vermischung einer Monomerflüssigkeit mit einem Knochenzementpulver durch mechanisches Vermischen, beispielsweise mittels eines Mischstabes.

In der Patentschrift EP 3 320 870 B1 wird im Gegensatz zu den vorgenannten Full-Prepacked-Systemen eine Vorrichtung beschrieben, bei welcher die Vermischung einer Monomerflüssigkeit mit einem Knochenzementpulver lediglich durch Einpressen der Monomerflüssigkeit in, insbesondere verdichtetes, Knochenzementpulver erfolgt. Die beschriebene Vorrichtung kommt daher ohne mechanisches Vermischen, insbesondere ohne einen Mischstab, aus. Derartige Vorrichtungen sind infolgedessen mischeinrichtungsfrei ausgestaltet.

In der Vorrichtung ist ein mit Monomerflüssigkeit gefüllter Behälter axial hinter einem mit einem Knochenzementpulver gefülltem Bereich innerhalb einer Kartusche lagert. Zwischen Knochenzementpulver und Behälter ist ein Austragskolben angeordnet. Um einen Knochenzementteig bereitzustellen, wird ein Förderkolben, welcher auf einer dem Austragskolben gegenüberliegenden Seite des Behälters angeordnet ist, in Richtung des Austragskolbens vorgetrieben, wodurch es zu einem Öffnen des Behälters, insbesondere durch teilweises Zerbersten eines Behälters in Form einer Glasampulle in Behälterteilstücke, kommt. Die aus dem Behälter austretende Monomerflüssigkeit wird durch fortgeführtes Vortreiben des Förderkolbens in das Knochenzementpulver unter Ausbildung des Knochenzementteigs gefördert. Als Behälter sind neben Glasampullen auch mit Monomerflüssigkeit gefüllte Beutel genannt. Um das Öffnen der Behälter zu unterstützen, kann in einer Weiterbildung der Vorrichtung an einer dem Behälter zugewandten Seite des Austragskolbens eine Öffnungshilfe angeordnet sein.

Vergleichbare Vorrichtungen sind ebenso in den Patentschriften EP 3 320 869 B1 und EP 3 403 716 B1 beschrieben.

Bei diesen Vorrichtungen sind die Menge an Knochenzementpulver und Monomerflüssigkeit aufeinander abgestimmt, so dass die Monomerflüssigkeit ausreicht, das Knochenzementpulver vollständig zu benetzen. Eine unvollständige Benetzung hätte negative Auswirkungen auf die Qualität und Homogenität des Knochenzementteigs.

Bei der Verwendung von Beuteln als Behälter für die Monomerflüssigkeit gibt es allerdings Einschränkungen. Diese Beutel sind üblicherweise aus Mehrschichtverbundfolien mit einer EVOH-Sperrschicht (Ethylen-Vinylalkohol-Sperrschicht), optional umfassend eine Metallbeschichtung, insbesondere umfassend eine Aluminiumbeschichtung, gefertigt, wobei es aus transportrechtlichen Gründen lediglich zulässig ist, einen Beutel mit bis zu 30 ml Monomerflüssigkeit zu befüllen. Zum Bereitstellen größerer Mengen an Knochenzementteig kann es daher notwendig sein, zwei oder mehr Beutel, welche jeweils mit bis zu 30 ml Monomerflüssigkeit befüllt sind, zu verwenden.

Aus produktionstechnischen Gründen weisen alle mit Monomerflüssigkeit befüllten Beutel stets einen Gaseinschluss, wie beispielsweise Luft oder ein Schutzgas, insbesondere Stickstoff oder Argon, auf. Das derart in den Beuteln eingeschlossene Gas muss durch das, insbesondere verdichtete, Knochenzementpulver hindurch transferiert werden, bevor das Knochenzementpulver mit der Monomerflüssigkeit unter Ausbildung des Knochenzementteigs in Kontakt kommt. Ein in Kontakt kommen des in der Ausbildung befindlichen Knochenzementteigs mit in den Beuteln eingeschlossenen Gases würde ansonsten zu Lufteinschlüssen, wie beispielsweise Gasblasen, im Knochenzementteig führen, welche aus diesem aufgrund der nicht vorhandenen mechanischen Vermischung nicht mehr vor dem Austragen aus der Vorrichtung entfernt werden könnten. Gasblasen im Knochenzementteig haben negative Auswirkungen auf die mechanischen Eigenschaften des aus dem Knochenzementteig durch Aushärtung entstehenden Knochenzements, was sich nachteilig auf die Stabilität einer damit fixierten Gelenkendoprothese auswirkt.

Die Patentschriften EP 3 320 870 B1, EP 3 320 869 B1 und EP 3 403 716 B1 offenbaren nicht wie zwei oder mehr Beutel zu öffnen sind, ohne dass der bereitgestellt Knochenzementteig Lufteinschlüsse, wie beispielsweise Gasblasen, enthält.

EP 3 395 274 A1 offenbart eine Vorrichtung zum Bereitstellen eines Knochenzementteigs aus zwei Ausgangskomponenten, umfassend eine hohlzylinderförmige Kartusche mit einem Innenraum, wobei in einem proximalen Teil des Innenraums ein Knochenzementpulver als erste Ausgangskomponente und in einem distalen Teil des Innenraums ein Beutel enthaltend eine Monomerflüssigkeit als zweite Ausgangskomponente lagert, wobei zwischen dem Knochenzementpulver und dem Beutel ein axial im Innenraum beweglicher Austragskolben und auf einer dem Austragskolben axial gegenüberliegenden Seite des Beutels ein axial im Innenraum beweglicher Förderkolben angeordnet ist, wobei der proximale Teil und der distale Teil des Innenraums über ein Leitungsmittel fluidleitend miteinander verbunden sind, wobei der Austragskolben an einer dem Beutel zugewandten distalen Austragskolbenseite in vier gleichgroße Quadranten unterteilbar ist, wobei durch ein Vortreiben des Förderkolbens in Richtung des Austragskolbens der Beutel durch Öffnungsmittel zu öffnen ist und die Monomerflüssigkeit in das Knochenzementpulver zu fördern ist.

### Aufgaben

Eine Aufgabe der vorliegenden Erfindung ist es, einen oder mehrere der sich aus dem Stand der Technik ergebenen Nachteile zumindest teilweise zu überwinden.

Im Speziellen basiert die Erfindung auf dem Ziel, eine Vorrichtung zur Verfügung zu stellen, welche auch größere Mengen an Knochenzementteig, insbesondere unter Verwendung von zwei oder mehr mit Monomerflüssigkeit befüllten Beuteln, einfach, zuverlässig, schnell und ohne Gasblasen bereitstellen kann. Die Vorrichtung soll den Knochenzement ohne eine mechanische Durchmischung der Ausgangskomponenten bereitstellen. Die Vorrichtung soll weiterhin so ausgestaltet sein, dass der Anwender keine Montageschritte durchführen muss. Die Vorrichtung soll ohne extern angelegtes Vakuum in der Lage sein den Knochenzement bereitzustellen. Die Vorrichtung soll den bereitgestellten Knochenzement austragen können. Die Vorrichtung soll den bereitgestellten Knochenzement ohne Umbaumaßnahmen austragen können. Die Vorrichtung soll ohne Umbaumaßnahmen und ohne externe Gerätschaften, wie beispielsweise Schläuche, Vakuumquellen oder Auspressvorrichtungen, den Knochenzement bereitstellen und austragen können. Die Vorrichtung soll mit möglichst wenigen Arbeitsschritte bedient werden können, um Fehlerquellen durch den Anwender zu minimieren.

Es ist eine weitere Aufgabe der Erfindung, ein Verfahren bereitzustellen, mit dem Knochenzement aus zwei Ausgangskomponenten bereitgestellt werden kann, mittels dem mindestens ein Teil der bereits beschriebenen Aufgaben zumindest zum Teil gelöst wird.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche geleistet. Die abhängigen Ansprüche stellen bevorzugte Ausführungsformen bereit, die zur mindestens teilweisen Erfüllung mindestens einer der Aufgaben beitragen.

Gegenstand der Erfindung ist eine Vorrichtung zum Bereitstellen eines Knochenzementteigs aus zwei Ausgangskomponenten, umfassend
eine hohlzylinderförmige Kartusche mit einem Innenraum, wobei in einem proximalen Teil des Innenraums ein Knochenzementpulver als erste Ausgangskomponente und in einem distalen Teil des Innenraums mindestens zwei Beutel enthaltend eine Monomerflüssigkeit als zweite Ausgangskomponente lagert,
wobei zwischen dem Knochenzementpulver und den mindestens zwei Beuteln ein axial im Innenraum beweglicher Austragskolben und auf einer dem Austragskolben axial gegenüberliegenden Seite der mindestens zwei Beutel ein axial im Innenraum beweglicher Förderkolben angeordnet ist,
wobei der proximale Teil und der distale Teil des Innenraums über ein Leitungsmittel fluidleitend miteinander verbunden sind,
wobei der Austragskolben an einer den mindestens zwei Beuteln zugewandten distalen Austragskolbenseite in vier gleichgroße Quadranten unterteilbar ist,
wobei jeder der vier Quadranten mindestens ein Öffnungsmittel aufweist, so dass durch ein Vortreiben des Förderkolbens in Richtung des Austragskolbens die mindestens zwei Beutel durch die Öffnungsmittel zu öffnen, insbesondere konzentrisch zu öffnen, sind und die Monomerflüssigkeit in das Knochenzementpulver zu fördern ist.

In einer Ausführungsform der Vorrichtung umfassen die Öffnungsmittel eine Spitze, eine Schneide oder eine Spitze und eine Schneide, um die Beutel zu öffnen. Diese Ausführungsform ist eine zweite Ausführungsform der Erfindung, welche vorzugsweise von der ersten Ausführungsform der Erfindung abhängt.

In einer Ausführungsform der Vorrichtung sind die Öffnungsmittel von einer Längsachse des Austragskolbens mit einer Entfernung in einem Bereich von einem Fünftel, als Bruch 1/5, bis zu vier Fünftel, als Bruch 4/5, eines Radius der Austragskolbenseite beabstanded. Diese Ausführungsform ist eine dritte Ausführungsform der Erfindung, welche vorzugsweise von der ersten oder der zweiten Ausführungsform der Erfindung abhängt.

In einer Ausführungsform der Vorrichtung umfassen die Öffnungsmittel in jedem, insbesondere in jedem einzelnen, Quadranten mindestens ein inneres Öffnungsmittel und mindestens ein äußeres Öffnungsmittel, wobei jeweils die inneren Öffnungsmittel und jeweils die äußeren Öffnungsmittel in jedem Quadranten konzentrisch um die Längsachse des Austragskolbens angeordnet sind. Diese Ausführungsform ist eine vierte Ausführungsform der Erfindung, welche vorzugsweise von der dritten Ausführungsform der Erfindung abhängt.

In einer Ausführungsform der Vorrichtung umfassen die Öffnungsmittel ein Metall, ein Polymer oder eine Kombination der vorgenannten. Insbesondere bestehen die Konussegmente aus einem Polymer oder einem Metall oder einer Kombination der vorgenannten. Diese Ausführungsform ist eine fünfte Ausführungsform der Erfindung, welche vorzugsweise von einer der vorhergehenden Ausführungsformen der Erfindung abhängt.

In einer Ausführungsform der Vorrichtung umfasst das Leitungsmittel in jedem Quadranten, insbesondere in jedem einzelnen Quadranten, mindestens eine axial durch den Austragskolben verlaufende, fluidleitende Durchführung, um den proximalen Teil und den distalen Teil des Innenraums fluidleitend miteinander zu verbinden. Diese Ausführungsform ist eine sechste Ausführungsform der Erfindung, welche vorzugsweise von einer der vorhergehenden Ausführungsformen der Erfindung abhängt.

In einer Ausführungsform der Vorrichtung münden die Durchführungen auf der distalen Austragskolbenseite in eine in der distalen Austragskolbenseite verlaufende, insbesondere oberflächlich verlaufende, Nut, welche die Durchführungen fluidleitend miteinander verbindet.

Diese Ausführungsform ist eine siebte Ausführungsform der Erfindung, welche vorzugsweise von der sechsten Ausführungsform der Erfindung abhängt.

In einer Ausführungsform der Vorrichtung umfassen die Durchführungen in jedem einzelnen Quadranten mindestens eine innere Durchführung und eine äußere Durchführung, wobei die inneren Durchführungen in eine in der distalen Austragskolbenseite verlaufende, insbesondere oberflächlich verlaufende, innere Nut und die äußeren Durchführungen in eine in der distalen Austragskolbenseite verlaufende, insbesondere oberflächlich verlaufende, äußere Nut münden, wobei die innere Nut die inneren Durchführungen und die äußere Nut die äußeren Durchführungen fluidleitend miteinander verbindet. Diese Ausführungsform ist eine achte Ausführungsform der Erfindung, welche vorzugsweise von der sechsten Ausführungsform der Erfindung abhängt.

In einer Ausführungsform der Vorrichtung sind die innere Nut und die äußere Nut über eine in der distalen Austragskolbenseite verlaufende, insbesondere oberflächlich verlaufende, Verbindungsnut fluidleitend miteinander verbunden. Diese Ausführungsform der Erfindung ist eine neunte Ausführungsform der Erfindung, welche vorzugsweise von der achten Ausführungsform der Erfindung abhängt.

In einer Ausführungsform der Vorrichtung weist der Förderkolben auf einer dem Austragskoben zugewandten proximalen Förderkolbenseite eine Aufnahme auf, um beim Vortreiben des Förderkolbens in Richtung des Austragskolbens die Öffnungsmittel aufzunehmen. Diese Ausführungsform der Erfindung ist eine zehnte Ausführungsform der Erfindung, welche vorzugsweise von einer der vorhergehenden Ausführungsformen der Erfindung abhängt.

In einer Ausführungsform der Vorrichtung umfasst die Aufnahme Ausnehmungen, um beim Vortreiben des Förderkolbens in Richtung des Austragskolbens die Öffnungsmittel aufzunehmen. Diese Ausführungsform der Erfindung ist eine elfte Ausführungsform der Erfindung, welche vorzugsweise von der zehnten Ausführungsform der Erfindung abhängt.

In einer Ausführungsform der Vorrichtung umfasst die Aufnahme eine Elastomerschicht, um beim Vortreiben des Förderkolbens in Richtung des Austragskolbens die Öffnungsmittel aufzunehmen. Diese Ausführungsform der Erfindung ist eine zwölfte Ausführungsform der Erfindung, welche vorzugsweise von der zehnten Ausführungsform der Erfindung abhängt.

Eine dreizehnte Ausführungsform der Erfindung ist ein Verfahren zum Bereitstellen eines Knochenzementteigs aus zwei Ausgangskomponenten mittels einer Vorrichtung nach einer der vorhergehenden Ausführungsformen, umfassend die folgenden Schritte:
a. Vortreiben des Förderkolbens in Richtung des Austragskolbens unter Öffnen der mindestens zwei Beutel durch die Öffnungsmittel,
b. Fördern der Monomerflüssigkeit in den proximalen Teil des Innenraums unter Ausbildung des Knochenzementteigs.

In einer Ausführungsform des Verfahrens wird zum Vortreiben des Förderkolbens die Vorrichtung in eine Austragseinrichtung eingesetzt. Diese Ausführungsform ist eine vierzehnte Ausführungsform der Erfindung, welche vorzugsweise von der dreizehnten Ausführungsform der Erfindung abhängt.

In einer Ausführungsform des Verfahrens wird die Monomerflüssigkeit mit Hilfe eines hydrophilen Additivs in dem Knochenzementpulver verteilt. Diese Ausführungsform ist eine fünfzehnte Ausführungsform der Erfindung, welche vorzugsweise von der dreizehnten oder vierzehnten Ausführungsform der Erfindung abhängt.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Wert Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "Parallelität" nie exakt, sondern nur innerhalb gewisser fertigungstechnischer Fehlertoleranzen gegeben sein kann. Beispielsweise schließen "im Wesentlichen parallele Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%. Eine "im Wesentlichen vollständige Befüllung eines Volumens B" umfasst beispielsweise eine Befüllung von ≥95 bis ≤100 Volumen-% des Gesamtvolumens von B.

Die Begriffe "proximal" und "distal" dienen lediglich der Bezeichnung der räumlich entgegengesetzten Enden der Vorrichtung oder anderer Struktureinheiten der Vorrichtung und erlauben keinen Rückschluss auf die Orientierung in Bezug zu einem menschlichen Körper, beispielsweise eines Anwenders der Vorrichtung. "Distal zu ..." und "proximal zu..." oder ähnliche Formulierungen drücken entsprechend lediglich die räumliche Anordnung zweier Struktureinheiten der Vorrichtung zueinander aus.

### Ausführliche Beschreibung

Ein erster Gegenstand der Erfindung betrifft eine Vorrichtung zum Bereitstellen eines Knochenzementteigs aus zwei Ausgangskomponenten, umfassend eine hohlzylinderförmige Kartusche mit einem Innenraum, wobei in einem proximalen Teil des Innenraums ein Knochenzementpulver als erste Ausgangskomponente und in einem distalen Teil des Innenraums mindestens zwei Beutel enthaltend eine Monomerflüssigkeit als zweite Ausgangskomponente lagert,
wobei zwischen dem Knochenzementpulver und den mindestens zwei Beuteln ein axial im Innenraum beweglicher Austragskolben und auf einer dem Austragskolben axial gegenüberliegenden Seite der mindestens zwei Beutel ein axial im Innenraum beweglicher Förderkolben angeordnet ist,
wobei der proximale Teil und der distale Teil des Innenraums über ein Leitungsmittel fluidleitend miteinander verbunden sind,
dadurch gekennzeichnet, dass
der Austragskolben an einer den mindestens zwei Beuteln zugewandten distalen Austragskolbenseite in vier gleichgroße Quadranten unterteilbar ist,
wobei jeder der vier Quadranten mindestens ein Öffnungsmittel aufweist, so dass durch ein Vortreiben des Förderkolbens in Richtung des Austragskolbens die mindestens zwei Beutel durch die Öffnungsmittel zu öffnen, insbesondere konzentrisch zu öffnen, sind und die Monomerflüssigkeit in das Knochenzementpulver zu fördern ist.

Die den mindestens zwei Beuteln zugewandte distale Austragskolbenseite weist in jedem ihrer gleich großen Quadranten mindestens ein Öffnungsmittel auf. Die Vorrichtung, insbesondere die distale Austragskolbenseite, weist somit also mindestens vier Öffnungsmittel auf, welche derart angeordnet sind, dass die mindestens zwei Beutel zuverlässig und konzertiert, also zeitlich aufeinander abgestimmt, insbesondere im Wesentlichen gleichzeitig, durch Schieben der Beutel gegen die Öffnungsmittel zu öffnen sind. Dies ermöglicht ein konzertiertes Austreten des in den Beuteln befindlichen Gases, welches, zusammen mit einem im distalen Teil des Innenraums bereits vor dem Öffnen der Beutel vorhandenem Gas, durch fortgeführtes Vortreiben des Förderkolbens in Richtung des Austragskolbens über das Leitungsmittel und durch das Knochenzementpulver transferiert werden kann, bevor die in den Beuteln gelagerte Monomerflüssigkeit über das Leitungsmittel unter Ausbildung des Knochenzementteigs in das Knochenzementpulver gefördert wird. Dies ermöglicht die Bereitstellung eines Knochenzementteigs ohne Gasblasen. Würden die Beutel hingegen zeitlich stark versetzt geöffnet werden, bestünde das Risiko, dass die Ausbildung des Knochenzementteigs bereits vor dem Öffnen des letzten Beutels einsetzte, so dass im letzten Beutel befindliches Gas zu Lufteinschlüssen, wie beispielsweise Gasblasen, im sich ausbildenden Knochenzementteig führen würde.

Innerhalb des distalen Teils des Innenraums der Vorrichtung lagern mindestens zwei Beutel enthaltend eine Monomerflüssigkeit. Unter einem Beutel wird eine nicht-starre, weitestgehend flexible Lagermöglichkeit verstanden, welche die Monomerflüssigkeit hermetisch dicht und steril lagern kann und mittels Einwirkung eines Öffnungsmittels, beispielsweise durch Aufstechen, Aufschneiden oder Aufreißen, zu öffnen ist. Die Beutel können beispielsweise aus einer Mehrschichtverbundfolie, vorzugsweise aufweisend eine EVOH-Sperrschicht gefertigt sein. Optional können die Beutel eine Metallbeschichtung, insbesondere eine Aluminiumbeschichtung, aufweisen.

Um ein zuverlässiges und konzertiertes Öffnen der mindestens zwei Beutel zu gewährleisten, ist es bevorzugt, dass die Beutel nebeneinander, im gleichen Abstand zu den Öffnungsmitteln, im distalen Teil des Innenraums angeordnet sind, so dass durch das Vortreiben des Förderkolbens in Richtung des Austragskolbens die Beutel konzertiert, insbesondere im Wesentlichen gleichzeitig, gegen die Öffnungsmittel geschoben werden können.

Mit Monomerflüssigkeit gefüllte Beutel sind häufig nicht kugelförmig, sondern länglich mit einer Beutellängsachse ausgebildet. Um die mindestens zwei Beutel konzertiert gegen die Öffnungsmittel zu schieben und so zu öffnen, insbesondere konzertiert zu öffnen, ist es bevorzugt, die Beutel nebeneinander so anzuordnen, dass die Beutellängsachsen im Wesentlichen parallel zu einer Längsachse des Austragskolbens im distalen Teil des Innenraums verlaufen.

Unter einem Öffnungsmittel ist eine an der distalen Austragskolbenseite ausgeformte Struktureinheit oder Strukturuntereinheit zu verstehen, welche einen Beutel öffnen, insbesondere aufstechen, aufschneiden oder aufreißen, kann, wenn das Öffnungsmittel und der Beutel gegeneinandergeschoben werden. Um das Öffnen, insbesondere Aufstecken, Aufschneiden oder Aufreißen, zu erleichtern, ist es bevorzugt, dass die Öffnungsmittel mit einer im Vergleich zu einer Querschnittsfläche des Innenraums geringen Fläche gegen die Beutel pressbar sind, die Öffnungsmittel also im weitestgehenden Sinne "scharf" und/oder "spitz" sind. Je nach Beschaffenheit des Beutels und des Öffnungsmittels kann zum Öffnen des Beutels durch das Öffnungsmittel ein unterschiedlich hoher Krafteintrag notwendig sein. Insbesondere ist der Krafteintrag durch die Öffnungsmittel, im Vergleich zu einem benötigten Krafteintrag durch Öffnen der Beutel mittels zweier planarer Kolben, verringert.

Um die Beutel zuverlässig, kontrolliert und konzertiert zu öffnen, weisen die Öffnungsmittel eine axiale Erstreckung in Richtung des Förderkolbens auf, welche beispielsweise einen Wert in einem Bereich von 5 mm bis 15 mm, bevorzugt 5 mm bis 10 mm, weiter bevorzugt 6 mm bis 8 mm beträgt. Öffnungsmittel mit der beschriebenen axialen Erstreckung fungieren in einer Ausgestaltungsform der Vorrichtung zusätzlich als Abstandshalter für geöffnete Beutel zum Leitungsmittel, so dass ein Risiko einer Verstopfung des Leitungsmittels durch die geöffneten Beutel oder Beutelteilstücke reduziert wird.

Die Vorrichtung weist eine hohlzylinderförmige Kartusche auf. Unter einer hohlzylinderförmigen Kartusche ist ein rohrartiges Behältnis zu verstehen, welches einen Innenraum und eine den Innenraum umgebende Kartuschenwand aufweist. Der Querschnitt der Kartusche kann beliebige Formen annehmen. Aufgrund der einfachen Fertigung und der anwendungssichereren Verwendung der Vorrichtung ist der Querschnitt, und bevorzugt auch der Querschnitt des Innenraums, kreisförmig ausgestaltet. Dies erlaubt eine gute Handhabbarkeit für den Anwender und reduziert durch eine Abwesenheit von Kanten ein Risiko einer Verkeilung beweglicher Teile innerhalb der Vorrichtung. Erfindungsgemäß kann die Kartusche aus unterschiedlichsten Materialien oder Materialkombinationen bestehen. Beispiele kann die Vorrichtung aus einem Polymer bestehen. Bevorzugt handelt es sich bei dem Polymer um ein transparentes Polymer, da der Anwender auf diese Weise eine ordnungsgemäße Funktion der Vorrichtung während einer Verwendung optisch kontrollieren kann.

Zwischen dem Knochenzementpulver und dem Behälter ist ein axial im Innenraum beweglicher Austragskolben angeordnet. Der Austragskolben dient der räumlichen Trennung der mindestens zwei Beutel und dem Knochenzementpulver, so dass weder Knochenzementpulver vom proximalen Teil des Innenraums in den distalen Teil des Innenraums noch die Beutel vom distalen Teil des Innenraums in den proximalen Teil des Innenraums gelangen können. Insbesondere letzteres verhindert einen mit Beutel oder Beutelteilstücken durchsetzten Knochenzementteig, welcher gesundheitliche Risiken für den Patienten darstellen und ein ordnungsgemäßes Austragen des Knochenzementteigs aus der Vorrichtung beeinträchtigen könnte. Der Austragskolben dient weiterhin dem Austragen des bereitgestellten Knochenzementteigs aus der Vorrichtung. Dazu kann der Austragskolben aus seiner ursprünglichen Position in Richtung einer Austragsöffnung der Vorrichtung verbracht werden. Die Austragsöffnung befindet sich bevorzugt an einer dem Austragskolben axial gegenüberliegenden Seite des Knochenzementpulvers. Um Gas, insbesondere in den Beuteln gelagertes Gas, aus der Vorrichtung zu entfernen, insbesondere vor dem Ausbilden des Knochenzementteigs zu entfernen, ist es bevorzugt, dass die Austragsöffnung gasdurchlässig ausgestaltet ist. Beispielsweise kann die Austragsöffnung mit einem gasleitenden Verschluss, wie beispielsweise einem Stopfen, verschlossen sein, welcher zum Austragen des angemischten Knochenzementteigs aus der Austragöffnung entfernbar ist.

Die Vorrichtung weist einen axial im Innenraum beweglichen Förderkolben auf. Der Förderkolben ist auf der dem Austragskolben axial gegenüberliegenden Seite der Beutel innerhalb der Vorrichtung angeordnet. Durch ein Vortreiben, das heißt durch eine Relativbewegung des Förderkolbens in Richtung des Austragskolbens, welche den Abstand von Förderkolben und Austragskolben innerhalb des Innenraums verkürzt, kommt es zu einem Öffnen der Beutel durch die Öffnungsmittel.

Ein fortgesetztes Vortreiben des Förderkolbens in Richtung des Austragskolbens führt zu einem Fördern der aus den Beuteln ausgetretenen Monomerflüssigkeit aus dem distalen Teil des Innenraums der Vorrichtung über das Leitungsmittel in den proximalen Teil des Innenraums der Vorrichtung. Dadurch kommt es zu einem Inkontaktbringen von Knochenzementpulver und Monomerflüssigkeit unter Ausbildung eines Knochenzementteigs.

Die Vorrichtung weist ein Leitungsmittel auf, durch welches der distale Teil und der proximale Teil des Innenraums fluidleitend verbunden sind. Fluidleitend bedeutet, dass der distale Teil und der proximale Teil des Innenraums für Flüssigkeiten, insbesondere die Monomerflüssigkeit, und für Gase durchlässig verbunden sind. Um zu verhindern, dass Knochenzementpulver aus dem proximalen Teil in den distalen Teil des Innenraums und die mindestens zwei Beutel, insbesondere Beutelteilstücke, aus dem distalen Teil in den proximalen Teil des Innenraums über das Leitungsmittel gelangen können, ist das Leitungsmittel vorzugsweise mit einem Filtermittel, insbesondere einer Porenscheibe, beispielsweise aus gesinterten Polypropylenpartikeln, aus gesinterten oder verpressten Polyethylenfasern, aus Cellulosefilz oder aus Pappe, ausgestattet, welche das Leitungsmittel für Feststoffe undurchlässig ausgestaltet. In einer Variante der Vorrichtung ist in dem Austragskolben und/oder zwischen dem Austragskolben und der Innenwand des Innenraums zumindest ein Kanal als Leitungsmittel vorgesehen, durch die der distale Teil und der proximale Teil des Innenraums fluidleitend miteinander verbunden sind. Dabei kann in oder an einem oder beiden Enden des zumindest einen Kanals ein für das Knochenzementpulver undurchlässiger und für die Monomerflüssigkeit und Gase durchlässiger Filter, beispielsweise eine Porenscheibe, beispielsweise aus gesinterten Polypropylenpartikeln, aus gesinterten oder verpressten Polyethylenfasern, aus Cellulosefilz oder aus Pappe, angeordnet sein. In einer weiteren Variante der Vorrichtung ist das Leitungsmittel eine oder mehrere Leitungen, die außen an der Kartusche oder in der Kartuschenwand angeordnet ist oder sind und den distalen Teil und den proximalen Teil des Innenraums verbindet oder verbinden. Der Austragskolben wird in dieser Variante umgangen.

Durch das Vortreiben des Förderkolbens in Richtung des Austragskolbens wird erreicht, dass die Monomerflüssigkeit innerhalb des Innenraums der Kartusche über das Leitungsmittel vom distalen Teil des Innenraums in den proximalen Teil des Innenraums, in dem sich das Knochenzementpulver befindet, transferiert werden kann.

Eine Variante der Vorrichtung ist ausgestaltet, dass ein fortgeführtes Vortreiben des Förderkolbens in Richtung des Austragskolbens nach erfolgtem Fördern der Monomerflüssigkeit vom distalen Teil des Innenraums in den proximalen Teil des Innenraums, ein Vortreiben des Austragskolbens in Richtung der Austragsöffnung der Vorrichtung bewirkt. Auf diese Weise kann der durch Mischung von Knochenzementpulver und Monomerflüssigkeit bereitgestellte Knochenzementteig durch die Austragsöffnung aus der Vorrichtung ausgetragen werden. Hiermit wird auf einfache Weise erreicht, dass der Knochenzementteig mit dem gleichen Antrieb aus der Kartusche auszutreiben ist, der auch zum Öffnen der Beutel und zum Fördern der Monomerflüssigkeit verwendet wird, nämlich mit dem unidirektional angetriebenem Förderkolben.

Um ein ungewolltes Vortreiben des Austragskolbens in Richtung der Austragsöffnung zu verhindern, kann am Austragskolben ein Rastmittel angeordnet sein, so dass der Austragskolben zwischen dem proximalen Teil und dem distalen Teil des Innenraums mit der Kartusche, insbesondere mit der Kartuschenwand, rasten kann, wobei diese Rastung durch die beim Öffnen der Beutel auftretenden Kräfte und einen auf die Monomerflüssigkeit vom Förderkolben ausgeübten Druck beim Fördern der Monomerflüssigkeit in den proximalen Teil des Innenraums nicht zu lösen ist, aber durch einen unmittelbaren Druck des Förderkolbens und eventuell dazwischen befindlicher Beutel oder Beutelteilstücke auf den Austragskolben beziehungsweise auf die Öffnungsmittel wirkenden Druck lösbar ist.

Durch das Rastmittel wird erreicht, dass zunächst die Beutel durch Vortreiben des Förderkolbens geöffnet werden können und die daraus austretende Monomerflüssigkeit mit dem Förderkolben anschließend in den proximalen Teil des Innenraums der Kartusche, also in das Knochenzementpulver, gepresst werden kann, wobei der Austragskolben dabei relativ zur Kartusche und zum Innenraum seine ursprüngliche Position hält. Erst nachdem die Monomerflüssigkeit weitgehend in das Knochenzementpulver gepresst wurde, und somit der Knochenzementteig im proximalen Teil des Innenraums der Kartusche vorliegt, kann anschließend der Knochenzementteig mit dem Austragskolben aus dem proximalen Teil der Kartusche gedrückt werden. Die Kraft zur Lösung der Rastung ist also größer als die zur Öffnung der Beutel und dem Fördern der Monomerflüssigkeit über das Leitungsmittel in den proximalen Teil des Innenraums notwendige Kraft.

Um ein unbeabsichtigtes Öffnen der Beutel, beispielsweise beim Transport der Vorrichtung, zu verhindern, kann es bevorzugt sein, dass im distalen Teil des Innenraums zwischen den Öffnungsmitteln und den Beuteln eine Transportsicherung angeordnet ist. Die Transportsicherung kann beispielsweise aus einer Platte mit Perforationen für den späteren Durchtritt der Öffnungsmittel gebildet sein, die sich mittels bei Krafteinwirkung einknickenden Stegen an der distalen Austragskolbenseite beabstandet.

Es kann auch vorgesehen sein, dass die Vorrichtung ein Mittel zur Druckentlastung aufweist, mit dem nach dem Transfer der Monomerflüssigkeit in das Knochenzementpulver überschüssige, unter Druck stehende Monomerflüssigkeit, abgelassen werden kann.

Um ein konzertiertes, im Wesentlichen gleichzeitiges, Öffnen der Beutel zu ermöglichen, sollten alle Beutel konzertiert, im Wesentlichen gleichzeitig, gegen die Öffnungsmittel geschoben werden können. Dazu sind an der distalen Austragkolbenseite in jedem der vier Quadranten der distalen Austragskolbenseite mindestens eines der Öffnungsmittel angeordnet.

Die vier Quadranten der distalen Austragskolbenseite sind alle gleichgroß und bilden gemeinsam die vollständige distale Austragskolbenseite ab. Es gibt keinen Bereich der den Beuteln zugewandten distalen Austragskolbenseite, welcher nicht einem der Quadranten zuordenbar ist.

Die anspruchsgemäße Anzahl und Anordnung der Öffnungsmittel stellt sicher, dass die Beutel zuverlässig und konzertiert, im Wesentlichen gleichzeitig, geöffnet werden können.

Dabei ist es bevorzugt, dass die Beutel nebeneinander und parallel zur Längsachse des Austragskolbens angeordnet sind und eine im Wesentlichen gleich große axiale Erstreckung im distalen Teil des Innenraums aufweisen.

Die Öffnungsmittel können unterschiedlich ausgeformt sein, um die Beutel zu öffnen.

Eine Ausführungsform der Vorrichtung ist dadurch gekennzeichnet, dass die Öffnungsmittel eine Spitze, insbesondere eine nadelartige Spitze, eine Schneide oder eine Spitze, insbesondere eine nadelartige Spitze, und eine Schneide umfassen. Dabei kann die Schneide geradlinig und/oder gebogen, beispielsweise kreisbogenförmig, ausgestaltet sein. Die Schneide kann dabei beispielsweise eine Länge in einem Bereich von 1 mm bis 8 mm aufweisen.

In einer Ausgestaltungsform sind die einzelnen Öffnungsmittel, bis auf ihre Verbindung über die gedacht plane distale Austragskolbenseite, disjunkt voneinander ausgestaltet. In einer weiteren Ausgestaltungsform sind die Öffnungsmittel Teil einer gemeinsamen Struktur, beispielsweise als Spitzen einer sägezahnartigen strukturellen Erhebung der distalen Austragkolbenseite, ausgestaltet.

Die Öffnungsmittel und die distale Austragskolbenseite können einstückig oder einteilig ausgestaltet sein. Einstückig bedeutet, dass die Öffnungsmittel und die distale Austragskolbenseite aus einzelnen Bauteilen zusammengesetzt sind. Einteilig bedeutet, dass die Öffnungsmittel Ausprägungen der distalen Austragskolbenseite sind.

Innerhalb der Quadranten können die Öffnungsmittel an unterschiedlichen Positionen angeordnet sein, um ein zuverlässiges und konzertiertes Öffnen der Beutel zu gewährleisten.

Eine Ausführungsform der Vorrichtung ist dadurch gekennzeichnet, dass die Öffnungsmittel von der Längsachse des Austragskolbens mit einer Entfernung in einem Bereich von einem Fünftel bis zu vier Fünftel eines Radius des Austragskolbens beabstandet sind. In dieser Ausführungsform weisen die Öffnungsmittel einen Abstand von der Längsachse des Austragskolbens auf, welcher dem 0,2-fachen bis 0,8-fachen des Radius der distalen Austragskolbenseite entspricht. In einer bevorzugten Ausgestaltungsform weist der Austragskolben einen im Wesentlichen kreisrunden Querschnitt auf, so dass die Längsachse des Austragskolbens durch den Mittelpunkt des Querschnitts und durch den Kontaktpunkt aller vier Quadranten verläuft. Der Radius der distalen Austragskolbenseite entspricht einer Wegstrecke vom Mittelpunkt der distalen Austragskolbenseite bis zu einem Rand der distalen Austragskolbenseite. Maximal könnte ein Öffnungsmittel mit einer Entfernung von der Längsachse des Austragskolbens entfernt sein, welche dem Radius der distalen Austragskolbenseite entspricht. Um ein zuverlässiges und konzertiertes Öffnen der Beutel zu gewährleisten, sind die Öffnungsmittel von der Längsachse mit einer Entfernung entfernt, welche dem 0,2-fachen bis 0,8-fachen des Radius der distalen Austragskolbenseite entspricht.

In einer Ausführungsform der Vorrichtung weisen alle Öffnungsmittel die gleiche Entfernung von der Längsachse des Austragskolbens auf. In dieser Ausführungsform sind die Öffnungsmittel konzentrisch um die Längsachse des Austragskolbens angeordnet.

Jeder Quadrant kann eine Vielzahl an Öffnungsmitteln aufweisen.

Eine Ausführungsform der Vorrichtung ist dadurch gekennzeichnet, dass die Öffnungsmittel in jedem Quadranten mindestens ein inneres Öffnungsmittel und mindestens ein äußeres Öffnungsmittel umfassen. In jedem Quadranten sind also mindestens zwei Öffnungsmittel vorhanden. Dabei weisen die inneren Öffnungsmittel eine geringere Entfernung von der Längsachse des Austragskolbens als die äußeren Öffnungsmittel auf. Bevorzugt weisen jeweils die inneren Öffnungsmittel und jeweils die äußeren Öffnungsmittel die gleiche Entfernung von der Längsachse des Austragskolbens auf. Die inneren Öffnungsmittel und die äußeren Öffnungsmittel sind in dieser bevorzugten Ausgestaltungsform somit jeweils konzentrisch um die Längsachse des Austragskolbens angeordnet.

Die Öffnungsmittel können unterschiedliche Materialien umfassen oder aus unterschiedlichen Materialien bestehen.

Eine Ausführungsform der Vorrichtung ist dadurch gekennzeichnet, dass die Öffnungsmittel ein Metall, ein Polymer oder eine Kombination aus einem Metall und einem Polymer umfassen, insbesondere aus einem Metall, einem Polymer oder einem Metall und einem Polymer bestehen. Beispiele für Metalle sind Titan, Tantal, Aluminium, Eisen und Stahl, insbesondere Edelstahl. Beispiele für Polymere sind Polyamide, Polyamid-imide, Polyarylether, Polysulfone, Polyetherketone. Die Polymere sollten dabei mechanisch derart belastbar sein, dass ein Öffnen der Beutel zuverlässig gelingt.

Das Leitungsmittel kann unterschiedlich ausgestaltet sein, um den distalen Teil und den proximalen Teil des Innenraums zuverlässig, insbesondere auch nach dem Öffnen der Beutel, fluidleitend zu verbinden.

Da die Beutel nach dem Öffnen und im Zuge des Förderns der Monomerflüssigkeit aus dem distalen Teil in den proximalen Teil des Innenraums durch Vortreiben des Förderkolbens in Richtung des Austragskolbens zusammengedrückt werden, besteht ein Risiko durch ein Verstopfen des Leitungsmittels durch einen Beutel, insbesondere durch einen geöffneten Beutel oder ein Beutelteilstück. Ein verstopftes Leitungsmittel ist fluidleitend geschlossen oder ist nur derart gering fluidleitend geöffnet, dass die Monomerflüssigkeit nur sehr langsam, beispielsweise nicht innerhalb einer Minute, in das Knochenzementpulver gefördert werden kann.

Eine Ausführungsform der Vorrichtung ist daher dadurch gekennzeichnet, dass das Leitungsmittel in jedem Quadranten mindestens eine axial durch den Austragskolben verlaufende, fluidleitende Durchführung umfasst, um den distalen Teil und den proximalen Teil des Innenraums fluidleitend zu verbinden. Die mindestens vier Durchführungen verlaufen von der distalen Austragskolbenseite bis zu einer der distalen Austragskolbenseite axial entgegengesetzten proximalen Austragskolbenseite durch den Austragskolben. Da in jedem Quadranten mindestens eine Durchführung vorhanden ist, verringert sich das Risiko eines vollständigen Verstopfens durch Zusetzen aller Durchführungen des Leitungsmittels gleichzeitig durch die Beutel, insbesondere durch einen geöffneten Beutel oder ein Beutelteilstück. Fluidleitend bedeutet, analog der Beschreibung des Leitungsmittels, dass der distale Teil und der proximale Teil des Innenraums für Flüssigkeiten, insbesondere die Monomerflüssigkeit, und für Gase durchlässig verbunden sind. Um zu verhindern, dass Knochenzementpulver aus dem proximalen Teil in den distalen Teil des Innenraums und die mindestens zwei Beutel aus dem distalen Teil in den proximalen Teil des Innenraums über das Leitungsmittel gelangen können, sind die Durchführungen vorzugsweise mit einem Filtermittel, insbesondere einer Porenscheibe, beispielsweise aus gesinterten Polypropylenpartikeln, aus gesinterten oder verpressten Polyethylenfasern, aus Cellulosefilz oder aus Pappe, ausgestattet, welche die Durchführungen für Feststoffe undurchlässig ausgestaltet.

In einer Ausgestaltungsform weisen die Durchführungen alle die gleiche Entfernung von der Längsachse des Austragskolbens auf.

Um die Monomerflüssigkeit auch bei teilweisem Verstopfen des Leitungsmittels, beispielsweise bei Verstopfen einer oder zweier Durchführungen in einem beziehungsweise zwei Quadranten, nahezu vollständig und zuverlässig in den proximalen Teil des Innenraums fördern zu können, sind in einer Ausführungsform die Durchführungen untereinander auf der distalen Austragskolbenseite fluidleitend miteinander verbunden. Auf diese Weise kann die Monomerflüssigkeit zuverlässig von einer verstopften Durchführung zu einer fluidleitend geöffneten Durchführung geleitet werden.

Eine Ausführungsform der Vorrichtung ist dadurch gekennzeichnet, dass die Durchführungen auf der distalen Austragskolbenseite in eine in der distalen Austragskolbenseite verlaufende Nut münden, welche die Durchführungen fluidleitend miteinander verbindet. Eine Nut ist eine Vertiefung, insbesondere eine in einem Querschnitt halbkreisförmige oder rechteckige Vertiefung, in der distalen Austragskolbenseite, durch die die Monomerflüssigkeit von einer Durchführung zu einer weiteren Durchführung in einem anderen Quadranten fließen kann. Dabei ist es bevorzugt, dass die Nut in einer Aufsicht kreisförmig ausgestaltet ist, so dass die Monomerflüssigkeit von einer verstopften Durchführung in zwei unterschiedliche Richtungen zu weiteren Durchführungen geleitet werden kann. Die Nut weist vorzugsweise eine Nutbreite in einem Bereich von 1 mm bis 3 mm auf, so dass das Risiko eines Verstopfens der Nut durch einen Beutel, insbesondere durch einen geöffneten Beutel oder ein Beutelteilstück, verringert ist.

In einer Ausgestaltungsform weisen die Durchführungen alle die gleiche Entfernung von der Längsachse des Austragskolbens auf und die Nut ist kreisförmig und konzentrisch um die Längsachse des Austragskolbens ausgestaltet.

In jedem Quadranten der distalen Austragskolbenseite kann mehr als eine Durchführung angeordnet sein.

Eine Ausführungsform der Vorrichtung ist dadurch gekennzeichnet, dass die Durchführungen in jedem Quadranten mindestens eine innere Durchführung und eine äußere Durchführung umfassen, wobei die innere Durchführung eine geringere Entfernung von der Längsachse des Austragskolbens als die äußere Durchführung aufweist.

Bevorzugt sind jeweils die inneren Durchführungen und jeweils die äußeren Durchführungen fluidleitend miteinander verbunden. Dazu münden die inneren Durchführungen in eine in der distalen Austragskolbenseite verlaufende innere Nut und die äußeren Durchführungen in eine in der distalen Austragskolbenseite verlaufende äußere Nut.

Dabei ist es bevorzugt, dass die innere Nut und die äußere Nut jeweils in einer Aufsicht auf die distale Austragskolbenseite kreisförmig ausgestaltet sind, so dass die Monomerflüssigkeit von einer verstopften Durchführung in zwei unterschiedliche Richtungen zu weiteren Durchführungen geleitet werden kann. Die innere Nut und die äußere Nut weisen vorzugsweise eine Nutbreite in einem Bereich von 1 mm bis 3 mm auf, so dass das Risiko eines Verstopfens der Nuten durch einen Beutel, insbesondere durch einen geöffneten Beutel oder ein Beutelteilstück, verringert ist.

In einer Ausgstaltungsform weisen jeweils die inneren Durchführungen und jeweils die äußeren Durchführungen die gleiche Entfernung von der Längsachse des Austragskolbens auf und die innere Nut und die äußere Nut sind jeweils kreisförmig und konzentrisch um die Längsachse des Austragskolbens ausgestaltet.

Um das Risiko eines Verstopfens des Leitungsmittels weiter zu reduzieren, ist eine Ausführungsform der Vorrichtung dadurch gekennzeichnet, dass die innere Nut und die äußere Nut mindestens über eine in der distalen Austragskolbenseite verlaufende Verbindungsnut fluidleitend miteinander verbunden sind. Die innere Nut und die äußere Nut kann beispielsweise über zwei, drei, vier oder mehr Verbindungsnuten fluidleitend miteinander verbunden sein.

Die Verbindungsnut weist vorzugsweise eine Nutbreite in einem Bereich von 0,5 mm bis 3 mm auf, so dass das Risiko eines Verstopfens der Verbindungsnut durch einen Beutel, insbesondere durch einen geöffneten Beutel oder ein Beutelteilstück, verringert ist.

Eine Ausführungsform der Vorrichtung ist dadurch gekennzeichnet, dass der Förderkolben auf einer dem Austragskolben zugewandten proximalen Förderkolbenseite eine Aufnahme aufweist, um beim Vortreiben des Förderkolbens in Richtung des Austragskolbens die Öffnungsmittel aufzunehmen. Die Aufnahme erlaubt ein Aufnehmen der Öffnungsmittel in den Förderkolben, so dass der Austragskolben und der Förderkolben näher zusammenschiebbar sind als ohne die Aufnahme. Ohne die Aufnahme wären der Austragskolben und der Förderkolben durch die Öffnungsmittel beabstandet. Die Aufnahme erlaubt somit eine bessere Ausnutzung der in den Beuteln vorhandenen Monomerflüssigkeit zum Bereitstellen des Knochenzementteigs.

Die Aufnahme kann unterschiedlich ausgestaltet sein, um die Öffnungsmittel in den Förderkolben aufzunehmen.

Eine Ausführungsform der Vorrichtung ist dadurch gekennzeichnet, dass die Aufnahme Ausnehmungen umfasst, bevorzugt dass die Aufnahme aus Ausnehmungen besteht. Die Ausnehmungen stellen Vertiefungen in der proximalen Förderkolbenseite dar, in welche die Öffnungsmittel einschiebbar sind. Bevorzugt sind die Ausnehmungen so ausgestaltet, dass die Öffnungsmittel die Ausnehmungen im Wesentlichen vollständig ausfüllen. Die Ausnehmungen stellen in dieser bevorzugten Ausgestaltungsform eine Negativform der Öffnungsmittel dar. Eine Ausführungsform der Vorrichtung ist dadurch gekennzeichnet, dass die Aufnahme eine Elastomerschicht umfasst, bevorzugt dass die Aufnahme aus einer Elastomerschicht besteht. Die Elastomerschicht ist dabei elastisch ausgestaltet, so dass die Öffnungsmittel die Elastomerschicht beim Vortreiben des Förderkolbens in Richtung des Austragskolben abschnittsweise zusammendrücken und die nicht zusammengedrückten Abschnitte der Elastomerschicht möglichst vollständig einen Zwischenraum zwischen den Öffnungsmitteln ausfüllen, um die Monomerflüssigkeit möglichst vollständig in den proximalen Teil des Innenraums zu fördern.

Ein weiterer Gegenstand der Erfindung betrifft ein Verfahren zum Bereitstellen eines Knochenzementteigs aus zwei Ausgangskomponenten mittels einer Vorrichtung, insbesondere mittels einer Vorrichtung nach einer der vorhergehenden Ausführungsformen, umfassend die folgenden Schritte:
a. Vortreiben des Förderkolbens in Richtung des Austragskolbens unter Öffnen der mindestens zwei Beutel durch die Öffnungsmittel,
b. Fördern der Monomerflüssigkeit in den proximalen Teil des Innenraums unter Ausbildung des Knochenzementteigs.

Durch das Vortreiben des Förderkolbens werden die Beutel gegen die Öffnungsmittel gedrückt, bis es im Zuge des Vortreibens zu einem Öffnen, insbesondere durch Aufstecken, Aufschneiden oder Aufreißen, der Beutel durch die Öffnungsmittel kommt. Die daraufhin aus den Beuteln entweichende Monomerflüssigkeit kann in Folge in den proximalen Teil des Innenraums gefördert werden. Das ebenso aus den Beuteln austretende Gas kann, zusammen mit bereits vor dem Öffnen der Beutel im distalen Teil des Innenraums vorhandenem Gas, bereits zeitlich vor der Monomerflüssigkeit über das Leitungsmittel und durch das Knochenzementpulver gefördert werden, so dass bei der zeitlich danach eintretenden Ausbildung des Knochenzementteigs keine Gasanschlüsse auftreten. Um zuerst das Gas und danach die Monomerflüssigkeit durch das Leitungsmittel in den proximalen Teil des Innenraums zu fördern, wird die Vorrichtung beim Fördern bevorzugt so gehalten, dass der proximale Teil des Innenraums räumlich höher als der distale Teil des Innenraums gehalten wird. Dadurch sammelt sich das Gas räumlich über der Monomerflüssigkeit und wird bei einem forstgesetzten Vortreiben des Förderkolbens in Richtung des Austragskolbens vor der Monomerflüssigkeit durch das Leitungsmittel gefördert.

Der Förderkolben kann auf unterschiedliche Weisen in Richtung des Austragskolben in die Kartusche vorgetrieben werden. Beispielsweise kann ein Anwender der Vorrichtung den Förderkolben manuell, insbesondere über Krafteinwirkung auf eine Stange oder Achse, vortreiben. In einer weiteren Ausführungsform bilden die Kartusche und der Förderkolben zusammen ein Gewinde aus, über welche der Förderkolben in Richtung des Austragskolben in die Kartusche hineingeschraubt werden kann. Dabei weist vorzugsweise die Kartusche ein Innengewinde und der Förderkolben ein Außengewinde auf, welche form- und/oder kraftschlüssig zusammenwirken, um das Vortreiben des Förderkolbens zu ermöglichen.

In einer Ausführungsform des Verfahrens erfolgt das Vortreiben des Förderkolbens unter Einsatz eines mechanischen Hilfsmittels.

Eine Ausführungsform des Verfahrens ist dadurch gekennzeichnet, dass zum Vortreiben des Förderkolbens die Vorrichtung in eine Austragseinrichtung, insbesondere eine Austragspistole für Knochenzementteige, eingesetzt wird. Austragspistolen für Knochenzementteige sind dem Fachmann bekannt.

Mit dem Fördern der Monomerflüssigkeit aus dem distalen Teil in den proximalen Teil des Innenraums beginnt die Ausbildung des Knochenzementteigs aus den beiden Ausgangskomponenten. Vorzugsweise erfolgt dies unter einer möglichst gleichmäßen Durchmischung der beiden Ausgangskomponenten, um einen möglichst homogenen Knochenzementteig zu erhalten. Die Durmischung der beiden Komponenten kann auf unterschiedliche Weise erfolgen. In einer Ausführungsform des Verfahrens erfolgt das Durchmischen unter aktiver Mitwirkung des Anwenders der Vorrichtung, beispielsweise unter Schütteln der Vorrichtung oder durch Betätigung eines Mischelements im proximalen Teil des Innenraums, insbesondere eines Rührers.

Eine Ausführungsform des Verfahrens ist dadurch gekennzeichnet, dass die Monomerflüssigkeit mit Hilfe eines hydrophilen Additivs in dem Knochenzementpulver verteilt wird. Ein Vorteil ist, dass dies ohne aktive Beteiligung des Anwenders der Vorrichtung vonstattengeht, was mögliche Fehler des Anwenders beim Mischen vermeidet. Ein möglicher Fehler ist, dass der Anwender nicht über die gesamte Länge des proximalen Teils des Innenraums mischt, so dass Teile des Knochenzementpulvers nicht mit Monomerflüssigkeit benetzt werden. Ein weiterer Vorteil ist, dass die Vorrichtung dadurch einfacher und mit weniger beweglichen Bauteilen ausgestaltet werden kann, was sowohl das Risiko für Fehlfunktionen als auch die Herstellungskosten der Vorrichtung verringert.

Die Vorrichtung ist dadurch gekennzeichnet, dass diese einen Knochenzementteig aus zwei Ausgangskomponenten bereitstellt. Unter einem Knochenzementteig wird eine Substanz verstanden, die geeignet ist im Bereich der Medizintechnik eine stabile Verbindung zwischen künstlichen Gelenken, wie beispielsweise Hüft- und Kniegelenken, und Knochenmaterial zu erstellen. Durch Aushärtung wird aus einem Knochenzementteig ein Knochenzement. Bevorzugt handelt es sich bei diesen Knochenzementen um Polymethylmethacrylat-Knochenzemente (PMMA Knochenzemente). PMMA-Knochenzemente kommen schon lange in medizinischen Anwendungen zum Einsatz und gehen auf Arbeiten von Sir Charnley zurück (vgl. Charnley, J. Anchorage of the femoral head prosthesis of the shaft of the femur. J. Bone Joint Surg. 1960; 42, 28-30.). PMMA-Knochenzemente können dabei aus einem Knochenzementpulver als erster Ausgangskomponente und einer Monomerflüssigkeit als zweiter Ausgangskomponente hergestellt werden. Bei geeigneter Zusammensetzung können die beiden Ausgangskomponenten getrennt voneinander lagerstabil sein. Bei Inkontaktbringen der beiden Ausgangskomponenten entsteht durch Quellung der Polymerbestandteile des Knochenzementpulvers ein plastisch verformbarer Knochenzementteig. Dabei wird eine Polymerisation des Monomers durch Radikale eingeleitet. Mit fortschreitender Polymerisation des Monomers erhöht sich die Viskosität des Knochenzementteigs, bis dieser vollständig aushärtet.

Unter einem Knochenzementpulver wird ein Pulver verstanden, welches mindestens ein partikuläres Polymethylmethacrylat und/oder ein partikuläres Polymethylmethacrylat-Copolymer umfasst. Beispiele für Copolymere sind Styren und/oder Methylacrylat. In einer Ausgestaltungsform kann das Knochenzementpulver zusätzlich ein hydrophiles Additiv umfassen, welches die Verteilung der Monomerflüssigkeit innerhalb des Knochenzementpulvers unterstützt. In einer weiteren Ausgestaltungsform kann das Knochenzementpulver zusätzlich einen Initiator, welcher die Polymerisation einleitet, umfassen. In einer weiteren Ausgestaltungsform kann das Knochenzementpulvers zusätzlich einen Röntgenopaker umfassen. In noch einer weiteren Ausgestaltungsform kann das Knochenzementpulver zusätzlich pharmazeutisch aktive Substanzen, wie beispielsweise Antibiotika, umfassen.

Bevorzugt umfasst das Knochenzementpulver als hydrophiles Additiv mindestens ein partikuläres Polymethylmethacrylat und/oder ein partikuläres Polymethylmethacrylat-Copolymer, einen Initiator und einen Röntgenopaker oder besteht aus diesen Komponenten. Weiter bevorzugt umfasst das Knochenzementpulver mindestens ein partikuläres Polymethylmethacrylat und/oder ein partikuläres Polymethylmethacrylat-Copolymer, einen Initiator, einen Röntgenopaker und ein hydrophiles Additiv oder besteht aus diesen Komponenten. Am bevorzugtesten umfasst das Knochenzementpulver mindestens ein partikuläres Polymethylmethacrylat und/oder ein partikuläres Polymethylmethacrylat-Copolymer, einen Initiator, einen Röntgenopaker, ein hydrophiles Additiv und ein Antibiotikum oder besteht aus diesen Komponenten.

Erfindungsgemäß kann die Partikelgröße des partikulären Polymethylmethacrylat und/oder des partikulären Polymethylmethacrylat-Copolymers des Knochenzementpulvers der Siebfraktion kleiner 150 µm, bevorzugt kleiner 100 µm, entsprechen.

Erfindungsgemäß kann das hydrophile Additiv partikulär und/oder faserförmig ausgestaltet sein. In einer weiteren Ausgestaltungsform kann das hydrophile Additiv schwerlöslich, bevorzugt unlöslich, in Methylmethacrylat sein. In einer weiteren Ausgestaltungsform kann das hydrophile Additiv ein Aufsaugvermögen von mindestens 0,6 g Methylmethacrylat pro Gramm hydrophiles Additiv besitzen. In einer weiteren Ausgestaltungsform kann das hydrophile Additiv eine chemische Substanz mit mindestens einer OH-Gruppe aufweisen. Dabei kann bevorzugt vorgesehen sein, dass das hydrophile Additiv kovalent gebundene OH-Gruppen an seiner Oberfläche besitzt. Beispiele für solche bevorzugten hydrophilen Additive können Additive ausgewählt aus der Gruppe umfassend Cellulose, Oxycellulose, Stärke, Titandioxid und Siliziumdioxid sein, wobei pyrogenes Siliziumdioxid besonders bevorzugt ist. In einer Ausgestaltungsform kann die Partikelgröße des hydrophilen Additivs der Siebfraktion kleiner 100 µm, bevorzugt kleiner 50 µm und am bevorzugtesten kleiner 10 µm entsprechen. Das hydrophile Additiv kann in einer Menge von 0,1 bis 2,5 Gew.-% bezogen auf das Gesamtgewicht des Knochenzementpulvers enthalten sein.

Erfindungsgemäß kann der Initiator Dibenzoylperoxid enthalten oder aus Dibenzoylperoxid bestehen.

Erfindungsgemäß versteht man unter einem Röntgenopaker eine Substanz, welche es erlaubt, den Knochenzement auf röntgendiagnostischen Aufnahmen sichtbar zu machen. Beispiele für Röntgenopaker können Bariumsulfat, Zirkondioxid und Kalziumcarbonat umfassen. Erfindungsgemäß kann die pharmazeutisch aktive Substanz ein oder mehrere Antibiotika und gegebenenfalls zugesetzte Co-Faktoren für das eine oder die mehreren Antibiotika umfassen. Bevorzugt besteht die pharmazeutisch aktive Substanz aus einem oder mehreren Antibiotika und gegebenenfalls zugesetzten Co-Faktoren für das eine oder die mehreren Antibiotika. Beispiele für Antibiotika sind unter anderem Gentamicin, Clindamycin und Vancomycin. Erfindungsgemäß kann die Monomerflüssigkeit das Monomer Methylmethacrylat umfassen oder aus Methylmethacrylat bestehen. In einer Ausgestaltungsform umfasst die Monomerflüssigkeit neben dem Monomer einen darin gelösten Aktivator, wie beispielsweise N,N-Dimethyl-p-toluidin, oder besteht aus Methylmethacrylat und N,N-Dimethyl-p-toluidin.

Die für die Vorrichtung offenbarten Merkmale sind auch für das Verfahren offenbart und umgekehrt.

### Figuren

Die Erfindung wird im Folgenden durch Figuren weiter beispielhaft illustriert. Die Erfindung ist nicht auf die Figuren beschränkt.

Es zeigen
- Fig. 1: einen schematischen Längsschnitt einer Vorrichtung zum Bereitstellen eines Knochenzementteigs umfassend zwei Beutel enthaltend eine Monomerflüssigkeit,
- Fig. 2: einen schematischen Längsschnitt eines Austragskolbens aus der Vorrichtung der Figur 1 in einer perspektivischen Seitenansicht,
- Fig. 3: eine perspektivische Seitenansicht des Austragskolbens aus den Figuren 1 und 2,
- Fig. 4: eine Aufsicht auf eine proximale Austragskolbenseite des Austragskolben aus den Figuren 1 bis 3,
- Fig. 5: eine Aufsicht auf eine distale Austragskolbenseite des Austragskolben aus den Figuren 1 bis 4,
- Fig. 6: einen schematischen Längsschnitt der Vorrichtung aus Figur 1 nach einem Öffnen der Beutel,
- Fig. 7: einen schematischen Längsschnitt der Vorrichtung aus den Figuren 1 und 6 beim Ausbilden des Knochenzementteigs,
- Fig. 8: einen schematischen Längsschnitt der Vorrichtung aus den Figuren 1, 6 und 7 beim Austragen des Knochenzementteigs,
- Fig. 9: einen schematischen Längsschnitt der Vorrichtung aus den Figuren 1 und 6 bis 8 mit einer Austragseinrichtung, und
- Fig. 10: ein Flussdiagramm eines Verfahrens zum Bereitstellen eines Knochenzementteigs.

### Beschreibung der Figuren

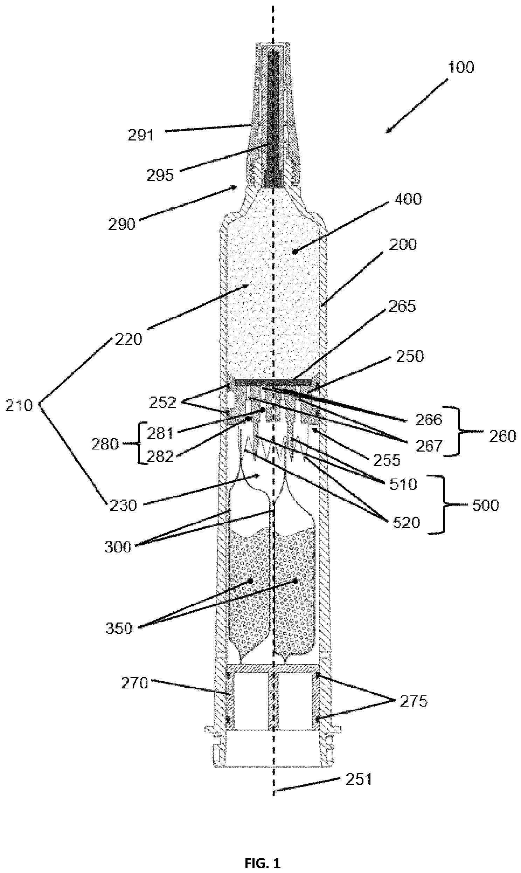

**Figur 1** zeigt einen schematischen Längsschnitt einer beispielhaften Ausführung einer Vorrichtung 100 zum Bereitstellen eines Knochenzementteigs in einem Ausgangszustand. Die Vorrichtung 100 ist einstückig, aber aus mehreren Bauteilen aufgebaut. Die Vorrichtung 100 ist rohrartig aufgebaut und umfasst eine hohlzylinderförmige Kartusche 200 mit einem Innenraum 210. In einem proximalen Teil 220 des Innenraums 210 ist ein Knochenzementpulver 400 als eine erste Ausgangskomponente und in einem distalen Teil 230 des Innenraums 210 sind zwei Beutel 300 enthaltend eine Monomerflüssigkeit 350 als zweite Ausgangskomponente des Knochenzementteigs gelagert. Neben der Monomerflüssigkeit 350 ist in den Beuteln 300 aus produktionstechnischen Gründen ein Gas gelagert. Das Knochenzementpulver 400 enthält als Hauptbestandteil partikuläres Polymethylmethacrylat sowie ein hydrophiles Additiv, mit dem die Monomerflüssigkeit 350 in dem Knochenzementpulver 400 mischeinrichtungsfrei verteilbar ist. Der proximale Teil 220 und der distale Teil 230 des Innenraums 210 sind durch einen axial im Innenraum 210 beweglichen Austragskolben 250 separiert. Der Austragskolben 250 ist für Feststoffe undurchlässig ausgestaltet, so dass kein Knochenzementpulver 400 vom proximalen Teil 210 in den distalen Teil 230 des Innenraums 210 und die Beutel 300 nicht vom distalen Teil 230 in den proximalen Teil 220 des Innenraums 210 gelangen.

Der Austragskolben 250 weist ein Leitungsmittel 260 in Form mehrerer Durchführungen auf, durch welches zwischen dem proximalen Teil 220 und dem distalen Teil 230 des Innenraums 210 eine fluidleitende Verbindung ausgebildet ist. Das Leitungsmittel 260 umfasst innere Durchführungen 266 und äußere Durchführungen 267, wobei die inneren Durchführungen 266 eine geringere Entfernung von einer Längsachse 251 des Austragskolbens 250 aufweisen als die äußeren Durchführungen 267.

Die inneren Durchführungen 266 und die äußeren Durchführungen 267 münden an einer den Beuteln 300 zugewandten distalen Austragskolbenseite 255 des Austragskolben 250 in kreisförmige, konzentrisch um die Längsachse 251 des Austragskolbens 250 verlaufende Nuten 280, wobei die inneren Durchführungen 266 in eine innere Nut 281 und die äußeren Durchführungen 267 in eine äußeren Nut 282 führen. Die Nuten 280 verbinden die jeweiligen Durchführungen fluidleitend miteinander, so dass die Monomerflüssigkeit 350 von einer durch die Beutel 300 nach deren Öffnen verstopften Durchführung zu einer fluidleitend geöffneten Durchführung fließen kann.

Das Leitungsmittel 260 ist durch eine Porenscheibe 265 für Feststoffe oder Knochenzementteige undurchlässig verschlossen, wobei die Porenscheibe aber ein problemloses Fördern der Monomerflüssigkeit 350 aus dem distalen Teil 230 in den proximalen Teil 220 des Innenraums 210 erlaubt. In der gezeigten Ausführungsform der Vorrichtung 100 ist die Porenscheibe 265 auf dem dem proximalen Teil 220 des Innenraums 210 zugewandten Ende des Leitungsmittels 260 angeordnet. In weiteren, nicht gezeigten, Ausführungsformen ist die Porenscheibe 265, oder andersartige Mittel, auf der dem distalen Teil 230 zugewandten Ende des Leitungsmittels 260 oder an beiden Enden des Leitungsmittels 260 angeordnet. Ein Vorteil einer wie gezeigt angeordneten Porenscheibe 265 ist, dass der sich im proximalen Teil 220 des Innenraums 210 ausbildende Knochenzementteig das Leitungsmittel 260 nicht verstopfen kann.

An der distalen Austragskolbenseite 255 des Austragskolbens 250 sind Öffnungsmittel 500 zum Öffnen der Beutel 300 angeordnet. Die Öffnungsmittel 250 umfassen innere Öffnungsmittel 510 und äußere Öffnungsmittel 520 (einige der äußeren Öffnungsmittel 520 hinter der Zeichenebene sind ebenso gezeigt, allerdings nicht beziffert), so dass die Beutel 300 zuverlässig und konzertiert öffenbar sind. Dazu sind die Beutel 300 im distalen Teil 230 des Innenraums 210 nebeneinander und entlang der Längsachse 251 des Austragskolbens 250 angeordnet, so dass beide Beutel 300 konzertiert gegen die Öffnungsmittel 250 pressbar sind. Auf der dem Austragskolben 250 gegenüberliegenden Seite der Beutel 300 ist ein innerhalb des Innenraums 210 axial beweglicher Förderkolben 270 angeordnet. Der Förderkolben 270 schließt eine Rückseite des Innenraums 210 der Kartusche 200 ab.

Damit aus den Beuteln 300 ausgetretene Monomerflüssigkeit 350 nicht am Austragskolben 250 vorbei in den proximalen Teil 220 des Innenraums 210 gedrückt wird, sind zwei radial umlaufenden Dichtungsringe 252 aus Gummi am Austragskolben 250 vorgesehen, mit denen der Austragskolben 250 gegen die Wand des Innenraums 210 abgedichtet ist. Ebenso sind am Förderkolben 270 zwei radial umlaufende Dichtungsringe 275 vorgesehen, mit denen ein Austritt der Monomerflüssigkeit 350 am Förderkolben 270 vorbei aus der Vorrichtung 100 heraus vermieden wird. Die Dichtungswirkung der Dichtungsringe 275 am Förderkolben 270 muss einem Druck widerstehen können, welcher durch den Förderkolben 270 auf die Monomerflüssigkeit 250 ausgeübt wird, um die Monomerflüssigkeit 350 nach dem Öffnen der Beutel 300 aus dem distalen Teil 230 in den proximalen Teil 220 des Innenraums 210 durch das Leitungsmittel 260 und die Porenscheibe 265 zu fördern.

Die Vorrichtung 100 weist ferner eine Austragsöffnung 290 auf, die den dem Austragskolben 250 abgewandten Bereich des proximalen Teil 220 des Innenraums 210 der Kartusche 200 begrenzt. Die Austragsöffnung 290 ist im Ausgangszustand der Vorrichtung 100 durch eine Verschlusskappe 291 mit einen Stopfen 295 verschlossen, so dass kein Knochenzementpulver 400 aus der Kartusche 200 entweichen kann. Der Stopfen 295 ist gasdurchlässig ausgestaltet, um ein Gas, welches aus den Beuteln 300 nach einem Öffnen der Beutel 300 entweicht und welches im distalen Teil 230 des Innenraums bereits vor dem Öffnen der Beutel 300 vorhanden ist, durch das Knochenzementpulver 400 hindurch und aus der Vorrichtung 100 heraus transferieren zu können, bevor die Ausbildung des Knochenzementteigs einsetzt.

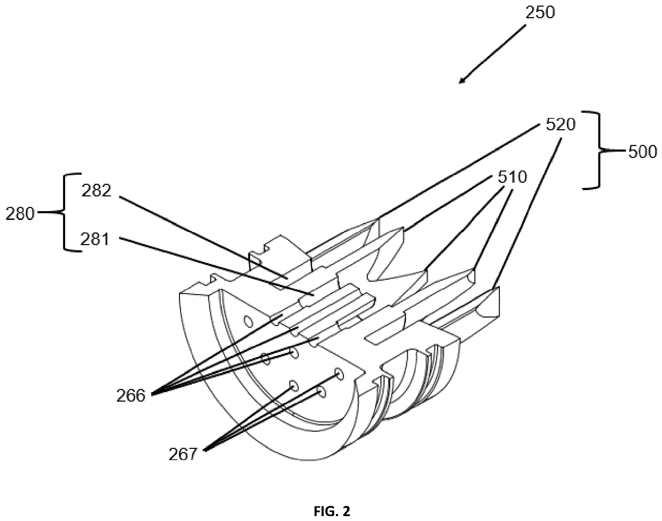

**Figur 2** zeigt den Längsschnitt des Austragskolben 250 der Vorrichtung 100 aus Figur 1 in einer perspektivischen Seitenansicht. Zur Vermeidung von Wiederholungen wird auf die vorstehende Beschreibung der Figur 1 verwiesen. In Figur 2 ist ersichtlich, dass die Öffnungsmittel 500 eine Schneide und/oder eine Spitze umfassen, um die Beutel 300 der Figur 1 zu öffnen, insbesondere die Beutel 300 der Figur 1 durch Aufstechen und/oder Aufschneiden zu öffnen.

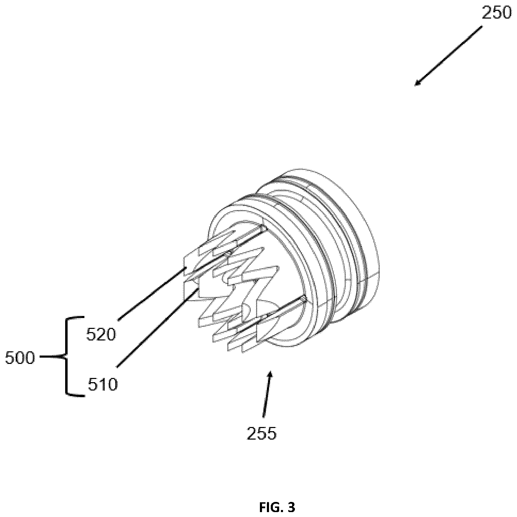

**Figur 3** zeigt den Austragskolben 250 der Figuren 1 und 2 in einer weiteren perspektivischen Seitenansicht. In Figur 3 ist ersichtlich, dass der Austragskolben 250 eine im Wesentlichen kreisrunde distale Austragskolbenseite 255 aufweist, welche in vier gleich große Quadranten unterteilbar ist. In jedem Quadranten sind mehrere innere Öffnungsmittel 510 und äußere Öffnungsmittel 520 (jeweils nur exemplarisch beziffert), welche zusammen die Öffnungsmittel 500 des Austragskolbens 250 bilden.

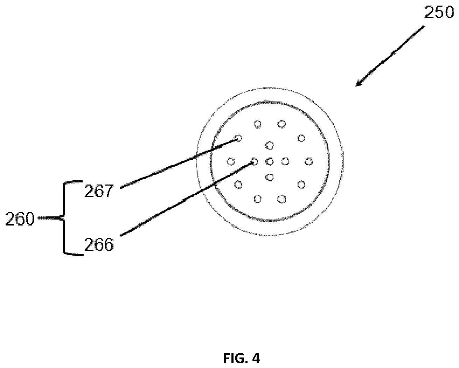

**Figur 4** zeigt den Austragskolben 250 der Figuren 1 bis 3 in einer Aufsicht auf eine proximale Austragskolbenseite. Die proximale Austragskolbenseite des Austragskolbens 250 ist in der Vorrichtung 100 der Figur 1 dem Knochenzementpulver 400 zugewandt. In Figur 4 ist ersichtlich, dass das Leitungsmittel 260 in jedem Quadranten des Austragskolbens 250 mehrere innere Durchführungen 266 und mehrere äußere Durchführungen 267 (jeweils nur exemplarisch beziffert) umfasst, so dass die Monomerflüssigkeit 350 der Figur 1 sicher und zuverlässig von der distalen Austragskolbenseite 255 auf die proximale Austragskolbenseite gefördert werden kann. Die inneren Öffnungsmittel 266 und die äußeren Durchführungen 267 sind jeweils konzentrisch um die Längsachse des Austragskolbens 250 (in Figur 4 nicht eingezeichnet) angeordnet.

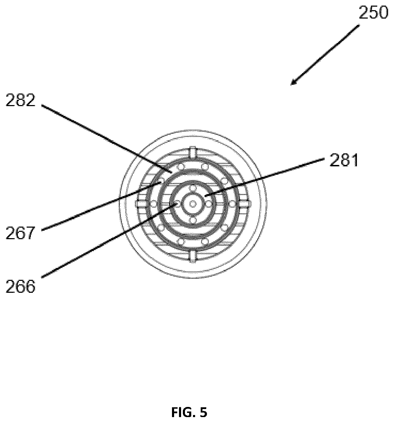

**Figur 5** zeigt den Austragskolben 250 der Figuren 1 bis 4 in einer Aufsicht auf die distale Austragskolbenseite 250. Die inneren Durchführungen 266 (nur exemplarisch beziffert) münden in die kreisförmige und konzentrisch um die Längsachse des Austragskolbens 250 (in Figur 5 nicht eingezeichnet) innere Nut 281, welche die inneren Durchführungen 266 fluidleitend miteinander verbindet. Die äußeren Durchführungen 267 (nur exemplarisch beziffert) münden in die kreisförmige und konzentrisch um die Längsachse des Austragskolbens 250 angeordnete äußere Nut 282, welche die äußeren Durchführungen 267 fluidleitend miteinander verbindet.

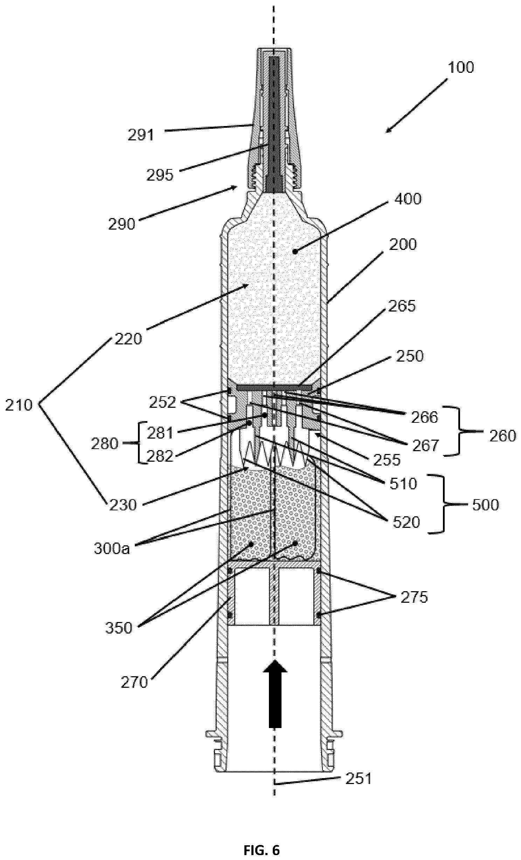

**Figur 6** zeigt die Vorrichtung 100 der Figur 1, wobei der Förderkolben 270 im Vergleich zu Figur 1 in Richtung des Austragskolbens 250 vorgetrieben ist (angedeutet durch einen Pfeil). Durch das Vortreiben des Förderkolbens 270 wurden die Beutel 300 der Figur 1 durch die Öffnungsmittel 500 geöffnet, wodurch die Monomerflüssigkeit 350 teilweise aus den geöffneten Beuteln 300a in den distalen Teil 230 des Innenraums 210 geflossen ist. Das aus den geöffneten Beuteln 300a in den distalen Teil 230 des Innenraums 210 ausgetretene Gas ist, mit bereits vor dem Öffnen der Beutel 300 im distalen Teil 230 des Innenraums 210 vorhandenem Gas zumindest teilweise durch das Vortreiben des Förderkolbens 270 in Richtung des Austragskolbens 250 durch das Leitungsmittel 260, das Knochenzementpulver 450 und den Stopfen 295 aus der Vorrichtung 100 heraus transferiert worden. Durch die besondere Ausgestaltungsform und Anordnung der Öffnungsmittel 500 wurden beide Beutel 300 der Figur 1 zuverlässig und konzertiert, im Wesentlichen gleichzeitig, geöffnet. Dies erlaubt ein nahezu vollständiges Entfernen des im distalen Teil 230 des Innenraums 210 vorhandenen Gases, bevor durch ein fortgesetztes Vortreiben des Förderkolbens 270 in Richtung des Austragskolbens 250 ein Fördern der Monomerflüssigkeit 350 über das Leitungsmittel 260 in das Knochenzementpulver 400 einsetzt. Dies verhindert Gasblasen im sich ausbildenden Knochenzementteig. Um zuerst das Gas und dann erst die Monomerflüssigkeit 350 in den proximalen Teil 220 des Innenraums 210 zu fördern, wird die Vorrichtung 100 beim Fördern bevorzugt so gehalten, dass der proximale Teil 220 räumlich höher als der distale Teil 230 des Innenraums 210 gehalten wird.

Das Vortreiben des Förderkolbens 270 kann auf unterschiedliche Weisen bewerkstelligt werden. Beispielsweise kann die Vorrichtung 100 dazu in eine Austragseinrichtung (nicht gezeigt) eingesetzt werden, welche den Förderkolben 270 mittels eines Stößels in Richtung des Austragskolbens 250 verbringt.

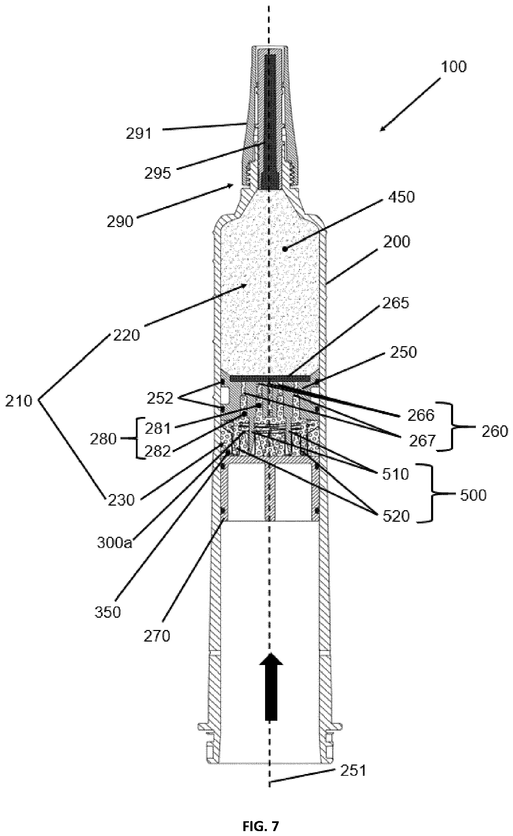

**Figur 7** zeigt die Vorrichtung 100 der Figuren 1 und 6, wobei der Förderkolben 270 im Vergleich zu Figur 6 weiter in Richtung des Austragskolbens 250 vorgetrieben ist (angedeutet durch einen Pfeil). Der Förderkolben 270 ist so weit in Richtung des Austragskolbens 250 vorgetrieben, dass der Förderkolben 270 in Kontakt mit den Öffnungsmitteln 500 steht. Durch das Vortreiben ist das zuvor im distalen Teil 230 des Innenraums 210 vorhandene Gas im Wesentlichen vollständig durch das Leitungsmittel 260 in den proximalen Teil 220 des Innenraums 210 und/oder teilweise durch den Stopfen 295 aus der Vorrichtung 100 heraus transportiert worden. Weiterhin ist die Monomerflüssigkeit 350, bis auf einen geringen Anteil in einem Zwischenraum der Öffnungsmittel sowie in den Nuten 280 und dem Leitungsmittel 260, zeitlich nach dem Gas durch das Leitungsmittel 260 in den proximalen Teil 220 des Innenraums 210 gefördert worden. Durch das Fördern hat sich aus dem Knochenzementpulver 400 der Figuren 1 und 6 sowie der Monomerflüssigkeit 350 ein im Wesentlichen blasenfreier Knochenzementteig 450 ausgebildet. Durch den Kontakt des Förderkolben 270 zu den Öffnungsmittel 500 kann der Förderkolben 270 nicht weiter in Richtung des Austragskolbens 250 vorgetrieben werden, ohne dass auch der Austragskolben 250 in Richtung der Austragsöffnung 290 verschoben wird. In einer weiteren, nicht gezeigten Ausführungsform der Vorrichtung 100 umfasst der Förderkolben 270 eine Aufnahme, welche ein Einschieben der Öffnungsmittel 500 in den Förderkolben 270 erlaubt. Durch die Aufnahme könnte weitere zwischen dem Austragskolben 250 und dem Förderkolben 270 gelagerte Monomerflüssigkeit 350 in den proximalen Teil 220 des Innenraums 230 gefördert werden, bevor ein Vortreiben des Austragskolbens 250 durch Vortreiben des Förderkolbens 270 einsetzt.

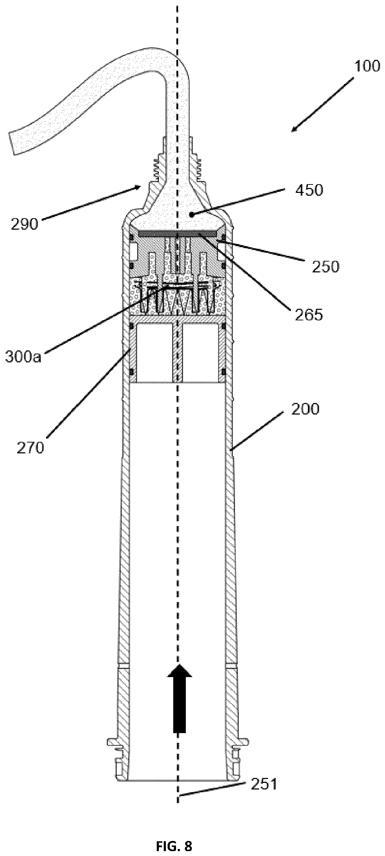

**Figur 8** zeigt die Vorrichtung 100 der Figuren 1, 6 und 7, wobei die Verschlusskappe 291 und der Stopfen 295 entfernt wurden und der Förderkolben 270, und damit auch der Austragskolben 250, im Vergleich zu Figur 7 weiter in Richtung der Austragsöffnung 290 vorgetrieben sind. Durch das Vortreiben des Austragskolbens 250 wurde der bereitgestellte Knochenzementteig 450 aus der Vorrichtung 100 teilweise ausgetragen. In einer weiteren, nicht gezeigten Ausführungsform der Vorrichtung 100 ist die Austragsöffnung 290 fluidleitend mit einem Austragsschnorchel verbunden, welche eine zielgenaue Applikation des Knochenzementteigs 450 an einer gewünschten Stelle ermöglicht.

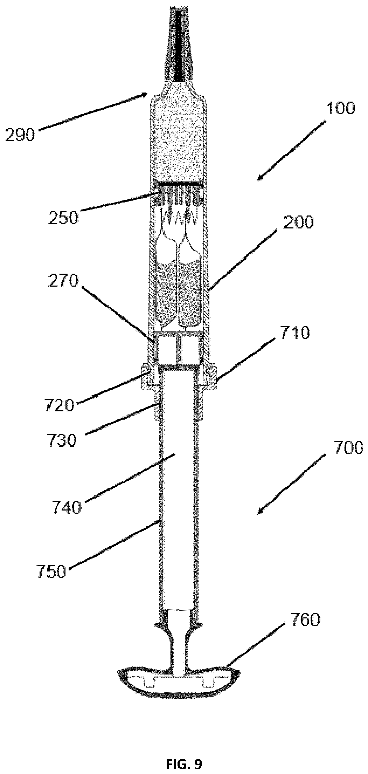

**Figur 9** zeigt die Vorrichtung 100 der Figuren 1 und 6 bis 8 mit einer Austragseinrichtung 700. Die Austragseinrichtung 700 ist an einem der Austragsöffnung 290 axial gegenüberliegendem Ende der Kartusche 200 über einen Bajonettverschluss 720 mit einem Verbindungselement 710 der Austragseinrichtung 700 verbunden. Das Verbindungselement 710 weist ein Innengewinde 730 auf, welches mit einem Außengewinde 750 eines Stößels 740 der Austragseinrichtung 750 form- und/oder kraftschlüssig zusammenwirkt, um den Stößel 740 mittels einer Drehbewegung durch das Verbindungselement 710 hindurch in die Kartusche 200 einzuschrauben. Um das Einschrauben des Stößels 740 für einen Anwender der Vorrichtung 100 zu erleichtern, weist die Austragseinrichtung 700 einen Griff 760 auf. Durch das Einschrauben des Stößels 740 in die Kartusche 200 wird ein Vortreiben des Förderkolbens 270 in Richtung des Austragskolbens 250 ausgelöst. In der gezeigten Ausführungsform der Austragseinrichtung 700 ist der Stößel 740 lang genug, um auch den mittels der Vorrichtung 100 bereitgestellten Knochenzementteig 450 (nicht gezeigt in Figur 9) aus der Austragsöffnung 290 auszutragen.

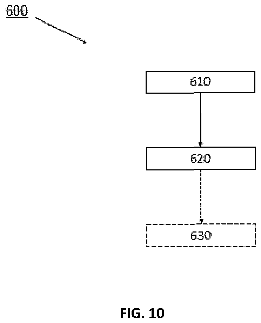

**Figur 10** zeigt einer Verfahren 600 zur Bereitstellung eines Knochenzementteigs 450 aus zwei Ausgangskomponenten mittels der Vorrichtung 100 gemäß den Figuren 1 und 6 bis 8 umfassend die Verfahrensschritte 610 und 620 und optional 630.

In einem Schritt 610 wird der Förderkolben 270 in Richtung des Austragskolbens 250 in die Kartusche 200 vorgetrieben. Durch das Vortreiben 610 werden die Beutel 300 gegen die Öffnungsmittel 500 gepresst und dadurch, insbesondere durch Aufstechen, Aufschneiden oder Aufreißen, zuverlässig und konzertiert, im Wesentlichen gleichzeitig, geöffnet. Aus den geöffneten Beuteln 300a kann die Monomerflüssigkeit 350 und das in den ungeöffneten Beuteln 300 gelagerte Gas in den distalen Teil 230 des Innenraums 210 austreten.

Im distalen Teil 230 des Innenraums 210 ist nach dem Öffnen der Beutel 300 die Monomerflüssigkeit 350 und Gas gelagert.

Um einen Knochenzementteig 450 mit möglichst wenig Gasblasen bereitzustellen, wird die Vorrichtung 100 vorzugsweise so gehalten, dass der Austragskolben 250 räumlich oberhalb des Förderkolbens 270 angeordnet ist. So wird aus dem distalen Teil 230 des Innenraums 210 zuerst dort vorhandenes Gas, wie beispielsweise Luft oder ein Schutzgas, insbesondere Stickstoff oder Argon, über das Leitungsmittel 260 in den proximalen Teil 220 des Innenraums 210 gefördert, bevor in einem Schritt 620 ein Fördern der Monomerflüssigkeit 350 durch das Leitungsmittel 260 unter Ausbildung des Knochenzementteigs 450 einsetzt. Auf diese Weise werden Gaseinschlüsse im Knochenzementpulver 400 durch die Monomerflüssigkeit 350 verdrängt und es erfolgt kein Einbringen weiteren Gases nach Beginn der Ausbildung des Knochenzementteiges 450.

In einem optionalen Schritt 630 wird der bereitgestellte Knochenzementteig 450 durch fortgeführtes Vortreiben des Förderkolbens 270 aus der Vorrichtung 100 durch die Austragsöffnung 290 im proximalen Teil 220 des Innenraums 210 ausgebracht. Dazu überwindet der Förderkolben 270 eine den Austragskolben 250 haltende Kraft, so dass Förderkolben 270 und Austragskolben 250 gemeinsam in Richtung der Austragsöffnung 290 innerhalb des Innenraums 210 verschoben werden und es zu einem Austragen des Knochenzementteigs 450 kommt.

Dadurch erfolgt das Öffnen der Beutel 300, das Fördern 620 der Monomerflüssigkeit 350 in den proximalen Teil 220 des Innenraums 210 und optional das Austragen 630 des bereitgestellten Knochenzementteigs 450 mit einer unidirektionalen linearen Bewegung des Förderkolbens 270 im Innenraum 210 der Kartusche 200.

Das Vortreiben 610 des Förderkolbens 270 kann durch manuelle Krafteinwirkung eines Anwenders der Vorrichtung 100 geschehen. In einer bevorzugten Ausführungsform des Verfahrens 600 wird zum Vortreiben 610 die Vorrichtung 100 in eine Austragseinrichtung 700 eingesetzt und der Förderkolben 270 durch Betätigung der Austragseinrichtung 700, beispielsweise einer Austragspistole, vorgetrieben. Dies erleichtert dem Anwender die Verwendung der Vorrichtung 100.

### Bezugszeichen

- 100: Vorrichtung
- 200: hohlzylinderförmige Kartusche
- 210: Innenraum der Kartusche
- 220: proximaler Teil des Innenraums
- 230: distaler Teil des Innenraums
- 250: Austragskolben
- 251: Längsachse des Austragskolbens
- 252: Dichtungsringe des Austragskolbens
- 255: distale Austragskolbenseite
- 260: Leitungsmittel
- 265: Porenscheibe
- 266: innere Durchführungen
- 267: äußere Durchführungen
- 270: Förderkolben
- 275: Dichtungsringe des Förderkolbens
- 280: Nut
- 281: innere Nut
- 282: äußere Nut
- 290: Austragsöffnung
- 291: Verschlusskappe
- 295: Stopfen
- 300: Beutel
- 300a: geöffneter Beutel
- 350: Monomerflüssigkeit
- 400: Knochenzementpulver
- 450: Knochenzementteig
- 500: Öffnungsmittel
- 510: innere Öffnungsmittel
- 520: äußeres Öffnungsmittel
- 600: Verfahren zur Bereitstellung eines Knochenzementteigs
- 610: Vortreiben
- 620: Fördern
- 630: Austragen
- 700: Austragseinrichtung
- 710: Verbindungselement
- 720: Bajonettverschluss
- 730: Innengewinde
- 740: Stößel
- 750: Außengewinde
- 760: Griff

## Patentansprüche

1. Vorrichtung (100) zum Bereitstellen eines Knochenzementteigs (450) aus zwei Ausgangskomponenten, umfassend
eine hohlzylinderförmige Kartusche (200) mit einem Innenraum (210), wobei in einem proximalen Teil (220) des Innenraums (210) ein Knochenzementpulver (400) als erste Ausgangskomponente und in einem distalen Teil (230) des Innenraums (210) mindestens zwei Beutel (300) enthaltend eine Monomerflüssigkeit (350) als zweite Ausgangskomponente lagert,
wobei zwischen dem Knochenzementpulver (400) und den mindestens zwei Beuteln (300) ein axial im Innenraum (210) beweglicher Austragskolben (250) und auf einer dem Austragskolben (250) axial gegenüberliegenden Seite der mindestens zwei Beutel (300) ein axial im Innenraum (210) beweglicher Förderkolben (270) angeordnet ist,
wobei der proximale Teil (220) und der distale Teil (230) des Innenraums (210) über ein Leitungsmittel (260) fluidleitend miteinander verbunden sind,
wobei
der Austragskolben (250) an einer den mindestens zwei Beuteln (300) zugewandten distalen Austragskolbenseite (255) in vier gleichgroße Quadranten unterteilbar ist,
wobei jeder der vier Quadranten mindestens ein Öffnungsmittel (500) aufweist, so dass durch ein Vortreiben des Förderkolbens (270) in Richtung des Austragskolbens (250) die mindestens zwei Beutel (300) durch die Öffnungsmittel (500) zu öffnen sind und die Monomerflüssigkeit (350) in das Knochenzementpulver (400) zu fördern ist.

2. Vorrichtung (100) nach Anspruch 1, wobei die Öffnungsmittel (500) eine Spitze und/oder eine Schneide umfassen, um die Beutel (300) zu öffnen.

3. Vorrichtung (100) nach Anspruch 1 oder 2, wobei die Öffnungsmittel (550) von einer Längsachse (251) des Austragskolbens (250) mit einer Entfernung in einem Bereich von einem Fünftel bis zu vier Fünftel eines Radius der distalen Austragskolbenseite (255) beabstandet sind.

4. Vorrichtung (100) nach Anspruch 3, wobei die Öffnungsmittel (250) in jedem Quadranten mindestens ein inneres Öffnungsmittel (510) und mindestens ein äußeres Öffnungsmittel (520) umfassen, wobei jeweils die inneren Öffnungsmittel (510) und jeweils die äußeren Öffnungsmittel (520) in jedem Quadranten konzentrisch um die Längsachse (251) des Austragskolbens (250) angeordnet sind.

5. Vorrichtung (100) nach einem der vorhergehenden Ansprüche, wobei die Öffnungsmittel (250) ein Metall oder ein Polymer umfassen.

6. Vorrichtung (100) nach einem der vorhergehenden Ansprüche, wobei das Leitungsmittel (260) in jedem Quadranten mindestens eine axial durch den Austragskolben (250) verlaufende, fluidleitende Durchführung umfasst, um den proximalen Teil (210) und den distalen Teil (220) des Innenraums (210) fluidleitend miteinander zu verbinden.

7. Vorrichtung (100) nach Anspruch 6, wobei die Durchführungen auf der distalen Austragskolbenseite (255) in eine in der distalen Austragskolbenseite (255) verlaufende Nut (280) münden, welche die Durchführungen fluidleitend miteinander verbindet.

8. Vorrichtung (100) nach Anspruch 6, die Durchführungen in jedem Quadranten mindestens eine innere Durchführung (266) und eine äußere Durchführung (267) umfassen, wobei die inneren Durchführungen (266) in eine in der distalen Austragskolbenseite (255) verlaufende innere Nut (281) und die äußeren Durchführungen (267) in eine in der distalen Austragskolbenseite (255) verlaufende äußere Nut (282) münden, wobei die innere Nut (281) die inneren Durchführungen (266) und die äußere Nut (282) die äußeren Durchführungen (267) fluidleitend miteinander verbindet.

9. Vorrichtung (100) nach Anspruch 8, wobei die innere Nut (281) und die äußere Nut (282) über eine in der distalen Austragskolbenseite (255) verlaufende Verbindungsnut fluidleitend miteinander verbunden sind.

10. Vorrichtung (100) nach einem der vorhergehenden Ansprüche, wobei der Förderkolben (270) auf einer dem Austragskolben (250) zugewandten proximalen Förderkolbenseite eine Aufnahme aufweist, um beim Vortreiben des Förderkolbens (270) in Richtung des Austragskolbens (250) die Öffnungsmittel (250) aufzunehmen.

11. Vorrichtung (100) nach Anspruch 10, wobei die Aufnahme Ausnehmungen umfasst.

12. Vorrichtung (100) nach Anspruch 10, wobei die Aufnahme eine Elastomerschicht umfasst.

13. Verfahren (600) zum Bereitstellen eines Knochenzementteigs (450) aus zwei Ausgangskomponenten mittels einer Vorrichtung (100) nach einem der vorhergehenden Ansprüche, umfassend die folgenden Schritte:
a. Vortreiben (610) des Förderkolbens (270) in Richtung des Austragskolbens (250) unter Öffnen der mindestens zwei Beutel (300) durch die Öffnungsmittel (500),
b. Fördern (620) der Monomerflüssigkeit (350) in den proximalen Teil (220) des Innenraums (210) unter Ausbildung des Knochenzementteigs (450).

14. Verfahren (600) nach Anspruch 13, wobei zum Vortreiben (610) des Förderkolbens (270) die Vorrichtung (100) in eine Austragseinrichtung (700) eingesetzt wird.

15. Verfahren (600) nach Anspruch 13 oder 14, wobei die Monomerflüssigkeit (350) mit Hilfe eines hydrophilen Additivs in dem Knochenzementpulver (400) verteilt wird.

## Claims

1. A device (100) for providing a bone cement paste (450) consisting of two starting components, comprising
a hollow cylindrical cartridge (200) having an interior (210), wherein a bone cement powder (400) as the first starting component is stored in a proximal part (220) of the interior (210) and at least two pouches (300) containing a monomer liquid (350) as the second starting component are stored in a distal part (230) of the interior (210), wherein a dispensing plunger (250) which is axially movable in the interior (210) is arranged between the bone cement powder (400) and the at least two pouches (300) and a delivery plunger (270) which is axially movable in the interior (210) is arranged on a side of the at least two pouches (300) axially opposite the dispensing plunger (250), wherein the proximal part (220) and the distal part (230) of the interior (210) are fluidically connected to one another via a conduit means (260),
wherein
the dispensing plunger (250) can be divided into four equally sized quadrants on a distal dispensing plunger side (255) facing the at least two pouches (300), wherein each of the four quadrants has at least one opening means (500), so that by advancing the delivery plunger (270) in the direction of the dispensing plunger (250), the at least two pouches (300) can be opened by the opening means (500) and the monomer liquid (350) can be delivered into the bone cement powder (400).

2. The device (100) according to claim 1, wherein the opening means (500) comprise a tip and/or a cutting edge for opening the pouches (300).

3. The device (100) according to either claim 1 or claim 2, wherein the opening means (550) are spaced apart from a longitudinal axis (251) of the dispensing plunger (250) at a distance in a range of from one fifth to four fifths of a radius of the distal dispensing plunger side (255).

4. The device (100) according to claim 3, wherein the opening means (250) in each quadrant comprise at least one inner opening means (510) and at least one outer opening means (520), wherein each of the inner opening means (510) and the outer opening means (520) are arranged in each quadrant concentrically about the longitudinal axis (251) of the dispensing plunger (250).

5. The device (100) according to any of the preceding claims, wherein the opening means (250) comprise a metal or a polymer.

6. The device (100) according to any of the preceding claims, wherein the conduit means (260) in each quadrant comprises at least one fluid-conducting feedthrough extending axially through the dispensing plunger (250) in order to fluidically connect the proximal part (210) and the distal portion (220) of the interior (210) to one another.

7. The device (100) according to claim 6, wherein the feedthroughs on the distal dispensing plunger side (255) open into a groove (280) extending in the distal dispensing plunger side (255), which groove fluidically connects the feedthroughs to one another.

8. The device (100) according to claim 6, wherein the feedthroughs in each quadrant comprise at least one inner feedthrough (266) and one outer feedthrough (267), wherein the inner feedthroughs (266) open into an inner groove (281) extending in the distal dispensing plunger side (255), and the outer feedthroughs (267) open into an outer groove (282) extending in the distal dispensing plunger side (255), wherein the inner groove (281) fluidically connects the inner feedthroughs (266) and the outer groove (282) fluidically connects the outer feedthroughs (267) to one another.

9. The device (100) according to claim 8, wherein the inner groove (281) and the outer groove (282) are fluidically connected to one another via a connecting groove extending in the distal dispensing plunger side (255).

10. The device (100) according to any of the preceding claims, wherein the delivery plunger (270) has a receptacle on a proximal delivery plunger side facing the dispensing plunger (250) in order to receive the opening means (250) when the delivery plunger (270) is advanced in the direction of the dispensing plunger (250).

11. The device (100) according to claim 10, wherein the receptacle comprises recesses.

12. The device (100) according to claim 10, wherein the receptacle comprises an elastomer layer.

13. A method (600) for providing a bone cement paste (450) consisting of two starting components by means of a device (100) according to any of the preceding claims, comprising the following steps:
a. advancing (610) the delivery plunger (270) in the direction of the dispensing plunger (250) to open the at least two pouches (300) by means of the opening means (500),
b. delivering (620) the monomer liquid (350) into the proximal part (220) of the interior (210) to form the bone cement paste (450).

14. The method (600) according to claim 13, wherein the device (100) is inserted into a dispensing apparatus (700) in order to advance (610) the delivery plunger (270).

15. The method (600) according to either claim 13 or claim 14, wherein the monomer liquid (350) is distributed in the bone cement powder (400) by means of a hydrophilic additive.

## Revendications

1. Dispositif (100) permettant de fournir une pâte de ciment osseux (450) à partir de deux composants de départ, comprenant
une cartouche (200) en forme de cylindre creux comportant un espace intérieur (210), dans lequel, dans une partie proximale (220) de l'espace intérieur (210), une poudre de ciment osseux (400) est stockée en tant que premier composant de départ et, dans une partie distale (230) de l'espace intérieur (210), au moins deux poches (300) contenant un liquide monomère (350) sont stockées en tant que second composant de départ,
dans lequel un piston d'évacuation (250) mobile axialement dans l'espace intérieur (210) est disposé entre la poudre de ciment osseux (400) et les au moins deux poches (300) et un piston de transport (270) mobile axialement dans l'espace intérieur (210) est disposé sur un côté des au moins deux poches (300) opposé axialement au piston d'évacuation (250), dans lequel la partie proximale (220) et la partie distale (230) de l'espace intérieur (210) sont reliées entre elles d'une manière conductrice de fluide par l'intermédiaire d'un moyen formant conduit (260),
dans lequel
le piston d'évacuation (250) peut être divisé en quatre quadrants de taille égale sur un côté distal de piston d'évacuation (255) faisant face aux au moins deux poches (300), dans lequel chacun des quatre quadrants présente au moins un moyen d'ouverture (500) de sorte que, par une avancée du piston de transport (270) en direction du piston d'évacuation (250), les au moins deux poches (300) doivent être ouvertes par les moyens d'ouverture (500) et le liquide monomère (350) doit être transporté dans la poudre de ciment osseux (400).

2. Dispositif (100) selon la revendication 1, dans lequel les moyens d'ouverture (500) comprennent une pointe et/ou une lame afin d'ouvrir les poches (300).

3. Dispositif (100) selon la revendication 1 ou 2, dans lequel les moyens d'ouverture (550) sont espacés d'un axe longitudinal (251) du piston d'évacuation (250) d'une distance dans une plage allant d'un cinquième à quatre cinquièmes d'un rayon du côté distal de piston d'évacuation (255).

4. Dispositif (100) selon la revendication 3, dans lequel les moyens d'ouverture (250) dans chaque quadrant comprennent au moins un moyen d'ouverture interne (510) et au moins un moyen d'ouverture externe (520), dans lequel respectivement les moyens d'ouverture internes (510) et respectivement les moyens d'ouverture externes (520) dans chaque quadrant sont disposés de manière concentrique autour de l'axe longitudinal (251) du piston d'évacuation (250).

5. Dispositif (100) selon l'une des revendications précédentes, dans lequel les moyens d'ouverture (250) comprennent un métal ou un polymère.

6. Dispositif (100) selon l'une des revendications précédentes, dans lequel le moyen formant conduit (260) comprend, dans chaque quadrant, au moins un passage conducteur de fluide s'étendant axialement à travers le piston d'évacuation (250) pour relier entre elles la partie proximale (210) et la partie distale (220) de l'espace intérieur (210) d'une manière conductrice de fluide.

7. Dispositif (100) selon la revendication 6, dans lequel les passages débouchent, sur le côté distal de piston d'évacuation (255), dans une rainure (280) s'étendant dans le côté distal de piston d'évacuation (255), laquelle rainure relie entre eux les passages d'une manière conductrice de fluide.

8. Dispositif (100) selon la revendication 6, dans lequel les passages dans chaque quadrant comprennent au moins un passage interne (266) et un passage externe (267), dans lequel les passages internes (266) débouchent dans une rainure interne (281) s'étendant dans le côté distal de piston d'évacuation (255) et les passages externes (267) débouchent dans une rainure externe (282) s'étendant dans le côté distal de piston d'évacuation (255), dans lequel la rainure interne (281) relie entre eux les passages internes (266) d'une manière conductrice de fluide et la rainure externe (282) relie entre eux les passages externes (267) d'une manière conductrice de fluide.

9. Dispositif (100) selon la revendication 8, dans lequel la rainure interne (281) et la rainure externe (282) sont reliées entre elles d'une manière conductrice de fluide par l'intermédiaire d'une rainure de liaison s'étendant dans le côté distal de piston d'évacuation (255).

10. Dispositif (100) selon l'une des revendications précédentes, dans lequel le piston de transport (270) présente un logement sur un côté proximal de piston de transport faisant face au piston d'évacuation (250) afin de recevoir les moyens d'ouverture (250) lors de l'avancée du piston de transport (270) en direction du piston d'évacuation (250).

11. Dispositif (100) selon la revendication 10, dans lequel le logement comprend des évidements.

12. Dispositif (100) selon la revendication 10, dans lequel le logement comprend une couche élastomère.

13. Procédé (600) permettant de fournir une pâte de ciment osseux (450) à partir de deux composants de départ à l'aide d'un dispositif (100) selon l'une des revendications précédentes, comprenant les étapes suivantes :
a. avancée (610) du piston de transport (270) en direction du piston d'évacuation (250) de façon à ouvrir les au moins deux poches (300) au moyen des moyens d'ouverture (500),
b. transport (620) du liquide monomère (350) dans la partie proximale (220) de l'espace intérieur (210) de façon à former la pâte de ciment osseux (450).

14. Procédé (600) selon la revendication 13, dans lequel, pour l'avancée (610) du piston de transport (270), le dispositif (100) est inséré dans un appareil d'évacuation (700).

15. Procédé (600) selon la revendication 13 ou 14, dans lequel le liquide monomère (350) est réparti dans la poudre de ciment osseux (400) à l'aide d'un additif hydrophile.
